(19) Europäisches Patentamt
European Patent Office
Office européen des brevets

(11) EP 0 703 222 A1

(12) **EUROPEAN PATENT APPLICATION**
published in accordance with Art. 158(3) EPC

(43) Date of publication:
**27.03.1996 Bulletin 1996/13**

(21) Application number: **94914613.8**

(22) Date of filing: **13.05.1994**

(51) Int. Cl.[6]: **C07D 223/16**, C07D 401/00, C07D 403/00, C07D 471/00, C07D 487/00, C07D 495/00, C07K 5/00, A61K 31/55, A61K 37/02

(86) International application number: **PCT/JP94/00774**

(87) International publication number:
**WO 94/26718 (24.11.1994 Gazette 1994/26)**

(84) Designated Contracting States:
**AT BE CH DE DK ES FR GB GR IE IT LI LU MC NL PT SE**

(30) Priority: **13.05.1993 JP 135312/93**

(71) Applicant: **YOSHITOMI PHARMACEUTICAL INDUSTRIES, LTD.**
**Osaka-shi Osaka 541 (JP)**

(72) Inventors:
- **TOMOZANE,Hideo,**
  **Brookline, MA 02146, USA (JP)**
- **MURAKAMI, Shu,**
  **c/o Yoshitomi Pharm. Ind., Ltd.**
  **Iruma-shi, Saitama 358 (JP)**
- **KITAJIMA, Hiroshi,**
  **c/o Yoshitomi Pharm. Ind., Ltd.**
  **Yoshitomimachi, Chikujo-gun,Fukuoaka 871 (JP)**
- **YASUMATSU, Hiroshi,**
  **Yoshitiomi Pharm. Ind., Ltd.**
  **Yoshitomimachi, Chikujo-gun, Fukuoka 871 (JP)**

(74) Representative: **von Kreisler, Alek, Dipl.-Chem. et al**
**Patentanwälte**
**von Kreisler-Selting-Werner**
**Postfach 10 22 41**
**D-50462 Köln (DE)**

(54) **3-AMINOAZEPINE COMPOUND AND PHARMACEUTICAL USE THEREOF**

(57) A 3-aminoazepine compound represented by general formula (I), an optically active isomer thereof, or a pharmaceutically acceptable salt thereof, wherein Ar represents phenyl, etc.; $R^1$ represents hydrogen, alkyl, etc.; $R^2$ and $R^3$ represent each independently hydrogen, alkyl, -CO-$R^6$ (wherein $R^6$ represents heteroaryl, etc.), -CONH-$R^7$ (wherein $R^7$ represents phenyl, etc.), etc.; $R^4$ represents hydrogen, alkyl, etc.; X represents oxygen or sulfur, provided $R^4$ and X may be combined together to form -C($R^{10}$)=N-N= (wherein $R^{10}$ represents hydrogen, alkyl, etc.). This compound has CCK and gastrin antagonisms and is useful as a remedy for central nervous system diseases such as anxiety and depression and as a preventive or a remedy for pancreatic diseases and gastrointestinal ulcer.

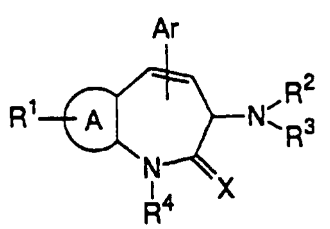

(I)



Printed by Rank Xerox (UK) Business Services
2.9.16/3.4

## Description

Technical Field

The present invention relates to novel 3-aminoazepine compounds having superior antagonistic action against cholecystokinin and gastrin, and pharmaceutically acceptable salts thereof.

Background Art

Cholecystokin (CCK) and gastrin are peptidergic hormones in gastrointestinal tract and control movements in the gastrointestinal tract and secretion of gastrointestinal juice. The two peptides have very similar chemical structures. That is, they both have a common C-terminal pentapeptide sequence. However, they express different actions in respective target tissues; gastrin mainly controls secretion of gastric acid and CCK, including CCK-58, CCK-33 and CCK-8 in biologically active forms, mainly controls contraction of gall bladder and secretion of pancreatic enzymes. CCK is widely present in brain as well and considered to function as a neurotransmitter or neuromodulater. The CCK present in the central nervous system is mostly CCK-8 and is either sulfuric acid type or desulfuric acid type. CCK acting at periphery (digestive tract) functions via a receptor subtype called CCK-A which is locally present at gall bladder, pancreas and intestinum ileum. The function of a CCK-A receptor found to be present in brain has not been elucidated. The central nervous action of CCK is mainly through a receptor subtype called CCK-B. The minimum agonist ligand thereof is a tetragastrin (CCK-4). A third receptor subtype is a gastrin receptor present in stomach. The gastrin receptor and CCK-B receptor are considered to be extremely similar. The main action at periphery via CCK-A receptor is gall bladder contraction and secretion of pancreatic enzyme. The role of CCK in the central nervous system via CCK-B receptor varies and the typical action thereof is anxiogenic action, panicking action, satiety action, pain action and control of dopamine nervous system. The action via gastrin receptor is gastric acid secretion.

Hence, the substance having an antagonistic action against these CCK and gastrin is effective for the prevention and treatment of various central nervous diseases such as anxiety, depression and schizophrenia, and various peripheral diseases such as pancreatic disorder and gastrointestinal ulcer, and there have been studied numerous antagonistic substances.

With regard to CCK antagonistic substance, Proc. Natl. Acad. Sci. USA, vol. 78, p 6304 (1981) and Eur. J. Med. Chem., vol. 21, p 9 (1986) report compounds represented by glutamic acid derivative represented by proglumide and lorglumide and aspartic acid derivative. However, these derivatives have problems in that they have weak CCK receptor affinity.

The benzodiazepine derivative having affinity for gastrin and CCK-B receptor, which is described in US Patent No. 4,820,834, J. Med. Chem., vol. 32, p 13 (1989) and WO92/11246 has been reported to have low bioavailability in J. Pharmacol. Exp. Ther., vol. 253, p 45 (1990). These derivatives are assumed to be unstable in vivo in view of the amidine structure they possess.

J. Med. Chem., vol. 34, p 404 (1991) describes $\alpha$-methyltriptophan derivative having affinity for CCK-B receptor. However, J. Med. Chem., vol. 36, p 2868 (1993) reports its low bioavailability (BA) that this derivative possesses.

J. Med. Chem., vol. 35, p 2534 (1992) describes quinazolinone derivative having affinity for CCK-B receptor.

In the 206th American Chemical International Symposium (1993), a pyrazolidinone derivative having affinity for CCK-B receptor was reported.

Japanese Patent Application under PCT laid-open under kohyo Nos. 504967/1993 and 504968/1993 describe glycineamide derivatives having affinity for CCK and gastrin receptor.

The compounds thus reported have problems in that they have low selectivity of receptor subtype, they are unstable in vivo, they have low effects, they have side-effects, they have low bioavailability or they have short duration of effects.

On the other hand, benzazepine compounds having hypotensive action (Japanese Patent Unexamined Publication No. 38074/1989), benzazepine compounds having brain function-improving action (Japanese Patent Unexamined Publication No. 250354/1989) and benzazepine compounds having CCK antagonistic action and gastrin antagonistic action (EP-A-487207) have been known as the compounds having an azepine skeleton. However, the 4-position carbon atom and the 5-position carbon atom are bonded by a single bond in these derivatives, and a substituent considered to be necessary for the expression of effects was pointed out. In International Application WO89/16524, benzazepine compounds having growth hormone release stimulating action has been disclosed.

In WO93/15059 which has been published after the priority date of the present application describes 3-phenylureido-2-oxo-5-phenyl-2,3,4,5-tetrahydro-1H-(1)-benzazepine derivatives having CCK-B antagonistic action. In these derivatives, the substituent considered to be necessary for the expression of effects cannot be located at a suitable stereoposition, since the 4-position carbon atom and 5-position carbon atom are bonded by a single bond. Moreover, increase of asymmetric carbon leads to problems in that production steps become complicated and isomers which may show side-effects increase. The 2-oxo-5-phenyl-2,3,4,5-tetrahydro-1H-(1)-benzazepine which is the starting material of the compound of the present invention is known in J. Med. Chem., vol. 14, pp 40-44 (1971).

As described supra, the CCK antagonists and gastrin antagonists heretofore reported do not have satisfactory effects and the development of a compound having more superior effects has been desired. Accordingly, the present invention aims at providing

1. a compound having a selective antagonistic action against either CCK-A receptor, CCK-B receptor or gastrin receptor,
2. a compound stable in vivo,
3. a compound having strong effects and less side-effects,
4. a compound having high bioavailability, and
5. a compound having long duration of effects.

Disclosure of the Invention

The present inventors have conducted intensive studies for the purpose of developing a more superior CCK antagonist and gastrin antagonist. That is, they designed a 3-aminoazepine structure so that they can avoid the unstable amidine structure of the 3-aminodiazepin skeleton to improve the stability in vivo of the compound, and linked the 4-position carbon atom and 5-position carbon atom of 3-aminoazepine skeleton by a double bond, so that the substituent considered to be necessary for the manifestation of effects can be located at a suitable stereoposition which is not achieved by a single bond [Bioorganic and Medicinal Chemistry Letters, vol. 3, pp 875-880 (1993)]. As a result, novel 3-aminoazepine compounds having high and selective affinity for CCK-A receptor, CCK-B receptor and gastrin receptor, as well as superior stability in vivo has been developed. The present invention has enabled elimination of the isomers posing problems of side-effects, by reducing asymmetric carbon atoms and provision of highly safe CCK antagonist and gastrin antagonist.

The 3-aminoazepine compound of the present invention shows good solubility, high intracerebral transition, high bioavailability and superior duration of effects.

That is, the present invention provides the following.

1. A 3-aminoazepine compound of the formula

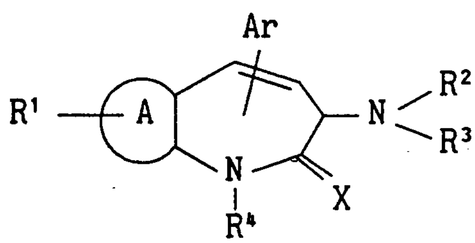

(I)

wherein

Ar is an optionally substituted aryl or an optionally substituted heteroaryl;

ring A is a benzene, a thiophene or a pyridine;

$R^1$ is a hydrogen, an alkyl, a cycloalkyl, a cycloalkylalkyl, an aralkyl, a hydroxy, an alkoxy, an amino, an alkylamino, a halogen or a group of the formula

$$-(CH_2)_kCOR^5$$

wherein k is 0 or an integer of 1-6 and $R^5$ is hydrogen, alkyl, cycloalkyl, cycloalkylalkyl, optionally substituted aryl, optionally substituted heteroaryl, aralkyl, hydroxy, alkoxy, amino, alkylamino, arylamino or aralkylamino;

$R^2$ and $R^3$ are the same or different and each is a hydrogen, an alkyl, a cycloalkyl, a cycloalkylalkyl, an aralkyl, a group of the formula

$$-(CH_2)_lC(=Y)-R^6$$

wherein l is 0 or an integer of 1-6, Y is oxygen atom or sulfur atom, $R^6$ is hydrogen, alkyl, cycloalkyl, cycloalkylalkyl, optionally substituted aryl, optionally substituted heteroaryl, aralkyl, hydroxy, alkoxy or amino, a group of the formula

$-C(=Y)-NH-R^7$

wherein Y is oxygen atom or sulfur atom, $R^7$ is alkyl, cycloalkyl, cycloalkylalkyl, optionally substituted aryl, optionally substituted heteroaryl or aralkyl, or a group of the formula

$-(CH_2)_mS(O)_n-R^8$

wherein m is 0 or an integer of 1-6, n is 0, 1 or 2 and $R^8$ is alkyl, cycloalkyl, cycloalkylalkyl, optionally substituted aryl, optionally substituted heteroaryl, aralkyl, hydroxy, alkoxy, amino, alkylamino, arylamino or aralkylamino, or $-N(R^2)(R^3)$ may be an amino group protected by a protecting group;

$R^4$ is a hydrogen, an alkyl, a cycloalkyl, a cycloalkylalkyl, an alkenyl, an aralkyl or a group of the formula

$-(CH_2)_pCOR^9$

wherein p is 0 or an integer of 1-6 and $R^9$ is hydrogen, alkyl, cycloalkyl, cycloalkylalkyl, optionally substituted aryl, optionally substituted heteroaryl, aralkyl, hydroxy, alkoxy, amino, alkylamino, arylamino, aralkylamino, or a cyclic amino of the formula

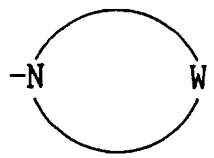

(a)

wherein W is $CH_2$, O or $N-R^{15}$ where $R^{15}$ is hydrogen, alkyl, acyl or benzyl; and

X is an oxygen atom or a sulfur atom, or $R^4$ and X may be bonded to each other to form a group of the formula $-C(R^{10})=N-N=$ where $R^{10}$ is hydrogen, alkyl, alkenyl, cycloalkyl, cycloalkylalkyl, adamantyl, aryl, aralkyl or a group of the formula

$-(CH_2)_qCOR^{11}$

wherein q is 0 or an integer of 1-6 and $R^{11}$ is hydrogen, alkyl, cycloalkyl, cycloalkylalkyl, aryl, heteroaryl, aralkyl, hydroxy, alkoxy, amino, alkylamino, arylamino or aralkylamino,

optically active compounds thereof and pharmaceutically acceptable salts thereof.

The preferable mode of the 3-aminoazepine compound of the formula (I) is the compounds of the formulas (Ia) - (Ie), with particular preference given to the compounds of the formulas (Ic) to (Ie).

2. A 3-aminoazepine compound of the formula

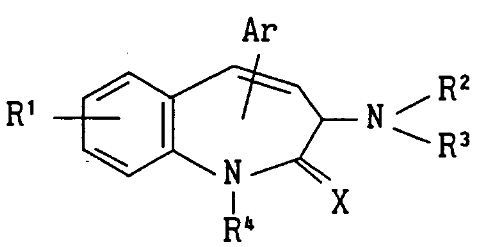

(Ia)

wherein each symbol is as defined in 1 above,
optically active compounds thereof and pharmaceutically acceptable salts thereof.

3. A 3-aminoazepine compound of the formula

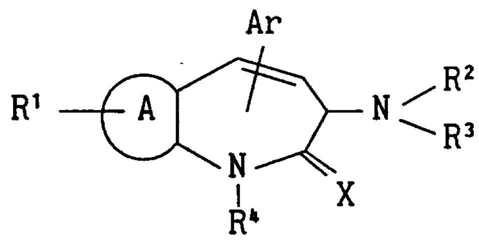

(Ib)

wherein A is a thiophene or a pyridine, and other symbols are as defined in 1 above, optically active compounds thereof and pharmaceutically acceptable salts thereof.

4. A 3-aminoazepine compound of the formula

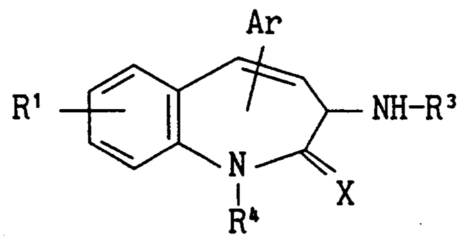

(Ic)

wherein

| | |
|---|---|
| Ar | is an optionally substituted aryl; |
| $R^1$ | is a hydrogen or an alkyl; |
| $R^3$ | is a group of the formula |

$$-(CH_2)_lC(=Y)-R^6$$

wherein l is 0 or an integer of 1-6, Y is oxygen atom or sulfur atom, $R^6$ is hydrogen, alkyl, cycloalkyl, cycloalkylalkyl, optionally substituted aryl, optionally substituted heteroaryl, aralkyl, hydroxy, alkoxy or amino, or a group of the formula

$$-C(=Y)-NH-R^7$$

wherein Y is oxygen atom or sulfur atom, $R^7$ is alkyl, cycloalkyl, cycloalkylalkyl, optionally substituted aryl, optionally substituted heteroaryl or aralkyl;

$R^4$ is a hydrogen, an alkyl or a group of the formula

$$-(CH_2)_pCOR^9$$

wherein p is an integer of 1-6 and $R^9$ is alkyl, cycloalkyl, cycloalkylalkyl, optionally substituted aryl, optionally substituted heteroaryl, aralkyl, hydroxy, alkoxy, amino, alkylamino, arylamino, aralkylamino, or a cyclic amino of the formula

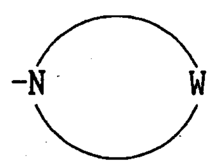

(a)

wherein W is $CH_2$, O or N-$R^{15}$ where $R^{15}$ is hydrogen, alkyl, acyl or benzyl; and

X is an oxygen atom, or $R^4$ and X may be bonded to each other to form a group of the formula: $-C(R^{10})=N-N=$ where $R^{10}$ is alkyl or cycloalkyl,

optically active compounds thereof and pharmaceutically acceptable salts thereof.

5. A 3-aminoazepine compound of the formula

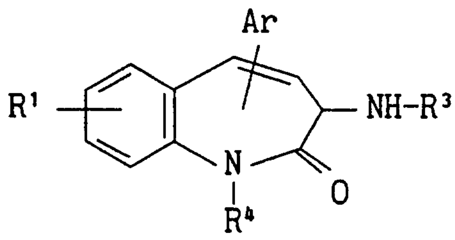

(Id)

wherein

Ar is an optionally substituted phenyl,
$R^1$ is a hydrogen or an alkyl;
$R^3$ is a group of the formula

$$-(CH_2)_lC(=Y)-R^6$$

wherein l is 0, Y is oxygen atom, $R^6$ is hydrogen, alkyl, cycloalkyl, cycloalkylalkyl, optionally substituted aryl, optionally substituted heteroaryl, aralkyl, hydroxy, alkoxy or amino, or a group of the formula

$$-C(=Y)-NH-R^7$$

wherein Y is oxygen atom, $R^7$ is alkyl, cycloalkyl, cycloalkylalkyl, optionally substituted aryl, optionally substituted heteroaryl or aralkyl; and
$R^4$ is an alkyl or a group of the formula

$$-(CH_2)_pCOR^9$$

wherein p is 1 and $R^9$ is optionally substituted aryl, optionally substituted heteroaryl, hydroxy, alkoxy, amino, alkylamino, arylamino, aralkylamino, or a cyclic amino of the formula

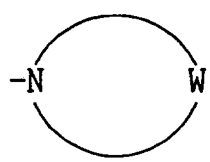

(a)

wherein W is $CH_2$, O or $N-R^{15}$ where $R^{15}$ is hydrogen, alkyl, acyl or benzyl,

optically active compounds thereof and pharmaceutically acceptable salts thereof.

6. A 3-aminoazepine compound of the formula

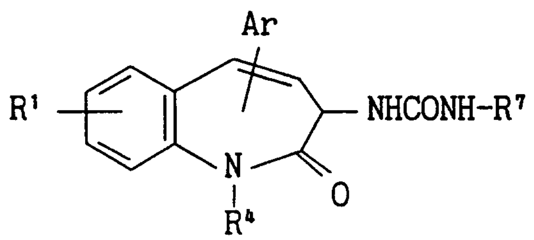

(Ie)

wherein

Ar is an optionally substituted phenyl;
$R^1$ is a hydrogen;
$R^7$ is an optionally substituted aryl or an optionally substituted heteroaryl; and
$R^4$ is a group of the formula

$$-(CH_2)_pCOR^9$$

wherein p is 1 and $R^9$ is optionally substituted aryl, hydroxy, alkoxy, alkylamino, or a cyclic amino of the formula

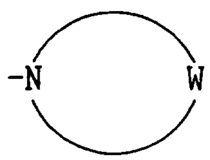

(a)

wherein W is $CH_2$, O or N-$R^{15}$ where $R^{15}$ is hydrogen, alkyl, acyl or benzyl,

optically active compounds thereof and pharmaceutically acceptable salts thereof.

In the above definitions and the present specification, the optionally substituted aryl represented by Ar is phenyl, 1-naphthyl, 2-naphthyl and the like, which may have, on its aromatic ring, 1 to 3 substituents selected from the group of halogen, alkyl having 1 to 4 carbon atoms, alkoxy having 1 to 4 carbon atoms, trifluoromethyl, nitro, amino, cyano and hydroxy. The optionally substituted heteroaryl is pyridyl (e.g., 2-pyridyl, 3-pyridyl and 4-pyridyl), quinolyl (e.g., 2-quinolyl and 3-quinolyl), indolyl (e.g., 2-indolyl and 3-indolyl), thienyl (e.g., 2-thienyl and 3-thienyl), furyl (e.g., 2-furyl and 3-furyl), benzofuranyl (e.g., 2-benzofuranyl and 3-benzofuranyl), 1H-benzoimidazol-2-yl, 2-benzothiazolyl and the like, which may have, on its ring, 1 to 3 substituents selected from the group of halogen, alkyl having 1 to 4 carbon atoms, alkoxy having 1 to 4 carbon atoms, trifluoromethyl, nitro, amino, cyano and hydroxy. Ar is aryl having substituent at the 5-position, with particular preference given to phenyl.

The benzene, thiophene or pyridine as the cyclic A denotes the following, with particular preference given to benzene.

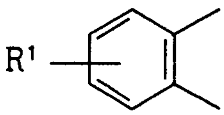 , 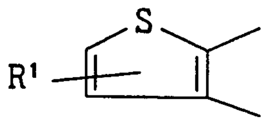 , 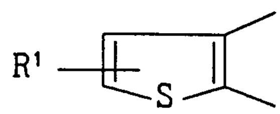

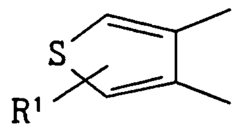 , 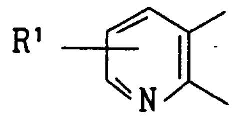 , 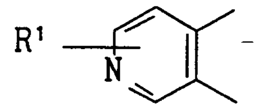

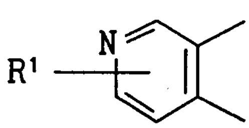 , 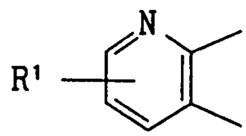

The alkyl is that having 1 to 20 carbon atoms, such as methyl, ethyl, propyl, isopropyl, butyl, isobutyl, tert-butyl, pentyl, isopentyl, tert-pentyl, hexyl, octyl, 2-ethylhexyl, 1,1,3,3-tetramethylbutyl, nonyl, decyl, dodecyl, tetradecyl, octadecyl and icosyl, with preference given to alkyl having 1 to 4 carbon atoms and particular preference given to methyl.

The cycloalkyl is that having 3 to 8 carbon atoms such as cyclopropyl, cyclobutyl, cyclopentyl, cyclohexyl, cycloheptyl and cyclooctyl, with preference given to cyclohexyl.

The cycloalkylalkyl is the above-mentioned alkyl substituted by cycloalkyl having 3 to 8 carbon atoms, and is exemplified by cyclopropylmethyl, cyclobutylmethyl, cyclopentylmethyl, cyclohexylmethyl, cycloheptylmethyl, cyclooctylmethyl, 2-cyclopentylethyl, 2-cyclohexylethyl and 3-cyclohexylpropyl.

The aralkyl is benzyl, 1-phenylethyl, 2-phenylethyl, 3-phenylpropyl, 4-phenylbutyl, 1-naphthylmethyl, 2-naphthylmethyl, diphenylmethyl and the like, which may have, on its aromatic ring, 1 to 3 substituents selected from the group of halogen, alkyl having 1 to 4 carbon atoms, alkoxy having 1 to 4 carbon atoms, trifluoromethyl, nitro, amino, cyano and hydroxy, with preference given to benzyl.

The alkoxy is that having 1 to 20 carbon atoms such as methoxy, ethoxy, propoxy, isopropoxy, butoxy, isobutoxy, tert-butoxy, pentyloxy, hexyloxy, octyloxy, decyloxy, dodecyloxy, octadecyloxy and icosyloxy, with preference given to alkoxy having 1 to 4 carbon atoms. Particularly preferred is methoxy.

The alkylamino is that having 1 to 8 carbon atoms such as methylamino, ethylamino, propylamino, isopropylamino, butylamino, isobutylamino, tert-butylamino, pentylamino, isopentylamino, hexylamino, heptylamino and octylamino, with preference given to alkylamino having 1 to 4 carbon atoms.

The halogen is chlorine, bromine, fluorine or iodine.

The optionally substituted aryl is phenyl or naphthyl, and may have 1 to 3 substituents selected from halogen, alkyl having 1 to 4 carbon atoms, alkoxy having 1 to 4 carbon atoms, trifluoromethyl, nitro, amino, cyano and hydroxy. The aryl at $R^7$ may have a substituent on its ring, such as carboxyalkyl (e.g., carboxymethyl, 2-carboxyethyl, 1-carboxyethyl, 1-carboxypropyl and 1-carboxybutyl, with preference given to carboxymethyl), alkoxycarbonylalkyl (e.g., methoxycarbonylmethyl, ethoxycarbonylmethyl, 2-methoxycarbonylethyl, 1-methoxycarbonylethyl, 1-methoxycarbonylpropyl and 1-methoxycarbonylbutyl, with preference given to methoxycarbonylmethyl) and 1H-tetrazol-5-yl. Preferred aryl is phenyl. Aryl at $R^7$ is preferably phenyl substituted by, for example, methyl, methoxy, halogen, carboxymethyl or 1H-tetrazol-5-yl.

The optionally substituted heteroaryl is, for example, pyridyl (e.g., 2-pyridyl, 3-pyridyl and 4-pyridyl), quinolyl (e.g., 2-quinolyl and 3-quinolyl), indolyl (e.g., 2-indolyl and 3-indolyl), thienyl (e.g., 2-thienyl and 3-thienyl), furyl (e.g., 2-furyl and 3-furyl), benzofuranyl (e.g., 2-benzofuranyl and 3-benzofuranyl), 1H-benzimidazol-2-yl or 2-benzothiazolyl, which may be substituted by 1 to 3 substituents selected from halogen, alkyl having 1 to 4 carbon atoms, alkoxy having 1 to 4 carbon atoms, trifluoromethyl, nitro, amino, cyano and hydroxy.

The heteroaryl at $R^6$, $R^7$ and $R^8$ may have a substituent on its ring, such as carboxyalkyl (e.g., carboxymethyl, 2-carboxyethyl, 1-carboxyethyl, 1-carboxypropyl and 1-carboxybutyl) and alkoxycarbonylalkyl (e.g., methoxycarbonylmethyl, ethoxycarbonylmethyl, 2-methoxycarbonylethyl, 1-methoxycarbonylethyl, 1-methoxycarbonylpropyl and 1-methoxycarbonylbutyl), and heteroaryl at $R^6$ is preferably indolyl (e.g., 2-indolyl and 3-indolyl). The heteroaryl at $R^7$ is preferably pyridyl or pyridyl substituted by methyl or methoxy.

The arylamino is, for example, phenylamino or naphthylamino, and may have 1 to 3 substituents selected from halogen, alkyl having 1 to 4 carbon atoms, alkoxy having 1 to 4 carbon atoms, trifluoromethyl, nitro, amino, cyano and hydroxy, with preference given to phenylamino.

The aralkylamino is, for example, benzylamino, 2-phenylethylamino, 3-phenylpropylamino or 4-phenylbutylamino, and may have 1 to 3 substituents selected from halogen, alkyl having 1 to 4 carbon atoms, alkoxy having 1 to 4 carbon atoms, trifluoromethyl, nitro, amino, cyano and hydroxy.

The amino protected by a protecting group is, for example, phthalimide, benzyloxycarbonylamino, tert-butoxycarbonylamino or triphenylmethylamino.

The alkenyl is that having 2 to 8 carbon atoms, such as vinyl, 1-propenyl, 2-propenyl, 2-pentenyl, 3-hexenyl and 6-octenyl.

When $R^5$, $R^6$, $R^9$ or $R^{11}$ is hydroxy, the compound of the formula (I) has a carboxyl group. The present invention embraces carboxylate which is easily hydrolyzed in the body and becomes free carboxylic acid or its salt, such as alkanoyloxyalkyl ester (e.g., acetoxymethyl, pivaloyloxymethyl, 1-acetoxyethyl and 1-pivaloyloxyethyl), alkoxycarbonyloxyalkyl ester (e.g., ethoxycarbonyloxymethyl and 1-ethoxycarbonyloxyethyl), ester (e.g., phthalidyl and dimethoxyphthalidyl), carbamoylalkyl ester (e.g., carbamoylmethyl, 2-carbamoylethyl, N-methylcarbamoylmethyl, N,N-dimethylcarbamoylmethyl and N,N-diethylcarbamoylmethyl), alkoxyalkyl ester (e.g., methoxymethyl and methoxyethyl) and 5-methyl-1,3-dioxolen-2-on-4-ylmethyl ester.

The cyclic amino represented by the formula (a) at $R^9$ is, for example, 1-pyrrolidinyl, piperidino, morpholino, 1-piperazinyl or 1-piperazinyl substituted by alkyl having 1 to 4 carbon atoms, acyl (e.g., acetyl, propionyl and benzoyl) or benzyl, at the 4-position.

With respect to the substituents for aryl, aralkyl, heteroaryl, aryl ring or heteroaryl ring, halogen means chlorine, fluorine, bromine or iodine, alkyl having 1 to 4 carbon atoms means methyl, ethyl, propyl, isopropyl, butyl, isobutyl or tert-butyl, with preference given to methyl, and alkoxy having 1 to 4 carbon atoms means methoxy, ethoxy, propoxy, isopropoxy, butoxy, isobutoxy and tert-butoxy, with preference given to methoxy.

The preferable compounds of the formula (I) are:
(+)-N-(1-methyl-2-oxo-5-phenyl-2,3-dihydro-1H-1-benzazepin-3-yl)-2-indolecarboxamide,
(+)-N-(1-ethoxycarbonylmethyl-2-oxo-5-phenyl-2,3-dihydro-1H-1-benzazepin-3-yl)-2-indolecarboxamide,
(+)-N-(1-(2'-methylphenacyl)-2-oxo-5-phenyl-2,3-dihydro-1H-1-benzazepin-3-yl)-2-indolecarboxamide,
(+)-N-(1-tert-butoxycarbonylmethyl-2-oxo-5-phenyl-2,3-dihydro-1H-1-benzazepin-3-yl)-2-indolecarboxamide,
(+)-N-(1-isopropoxycarbonylmethyl-2-oxo-5-phenyl-2,3-dihydro-1H-1-benzazepin-3-yl)-2-indolecarboxamide,
(+)-N-(1-tert-butylaminocarbonylmethyl-2-oxo-5-phenyl-2,3-dihydro-1H-1-benzazepin-3-yl)-2-indolecarboxamide,
(+)-N-(1-diethylaminocarbonylmethyl-2-oxo-5-phenyl-2,3-dihydro-1H-1-benzazepin-3-yl)-2-indolecarboxamide, and
(+)-N-(2-oxo-5-phenyl-1-(pyrrolidin-1-ylcarbonylmethyl)-2,3-dihydro-1H-1-benzazepin-3-yl)-2-indolecarboxamide, and these compounds show particularly strong affinity for CCK-A receptor.

The following compounds of the formula (I) are also preferred.
(-)-1-(1-ethoxycarbonylmethyl-2-oxo-5-phenyl-2,3-dihydro-1H-1-benzazepin-3-yl)-3-(3-methylphenyl)urea,
(-)-1-(1-ethoxycarbonylmethyl-2-oxo-5-phenyl-2,3-dihydro-1H-1-benzazepin-3-yl)-3-(4-methylphenyl)urea,
(-)-1-(1-ethoxycarbonylmethyl-2-oxo-5-phenyl-2,3-dihydro-1H-1-benzazepin-3-yl)-3-(3-methoxyphenyl)urea,
(-)-1-(1-(2'-methylphenacyl)-2-oxo-5-phenyl-2,3-dihydro-1H-1-benzazepin-3-yl)-3-(4-methylphenyl)urea,
(-)-1-(1-(2'-methylphenacyl)-2-oxo-5-phenyl-2,3-dihydro-1H-1-benzazepin-3-yl)-3-(2-methylphenyl)urea,
(-)-1-(4-methoxyphenyl)-3-(1-(2'-methylphenacyl)-2-oxo-5-phenyl-2,3-dihydro-1H-1-benzazepin-3-yl)urea,
(-)-1-(2-chlorophenyl)-3-(1-(2'-methylphenacyl)-2-oxo-5-phenyl-2,3-dihydro-1H-1-benzazepin-3-yl)urea,
(-)-1-(3-carboxymethylphenyl)-3-(1-(2'-methylphenacyl)-2-oxo-5-phenyl-2,3-dihydro-1H-1-benzazepin-3-yl)urea,
(-)-1-(3-carboxymethylphenyl)-3-(1-ethoxycarbonylmethyl-2-oxo-5-phenyl-2,3-dihydro-1H-1-benzazepin-3-yl)urea,
(-)-1-(1-tert-butoxycarbonylmethyl-2-oxo-5-phenyl-2,3-dihydro-1H-1-benzazepin-3-yl)-3-(3-methylphenyl)urea,
(-)-1-(3-methylphenyl)-3-(2-oxo-5-phenyl-1-isopropoxycarbonylmethyl-2,3-dihydro-1H-1-benzazepin-3-yl)urea,
(-)-1-(1-tert-butylaminocarbonylmethyl-2-oxo-5-phenyl-2,3-dihydro-1H-1-benzazepin-3-yl)-3-(3-methylphenyl)urea,
(-)-1-(3-methylphenyl)-3-(2-oxo-5-phenyl-1-(pyrrolidin-1-ylcarbonylmethyl)-2,3-dihydro-1H-1-benzazepin-3-yl)urea,
(-)-1-(2-methylpyridin-6-yl)-3-(2-oxo-5-phenyl-1-(pyrrolidin-1-ylcarbonylmethyl)-2,3-dihydro-1H-1-benzazepin-3-yl)urea,
(-)-1-(2-methoxypyridin-5-yl)-3-(2-oxo-5-phenyl-1-(pyrrolidin-1-ylcarbonylmethyl)-2,3-dihydro-1H-1-benzazepin-3-yl)urea,
(-)-1-(1-diethylaminocarbonylmethyl-2-oxo-5-phenyl-2,3-dihydro-1H-1-benzazepin-3-yl)-3-(3-methylphenyl)urea,
(-)-1-(1-diethylaminocarbonylmethyl-2-oxo-5-phenyl-2,3-dihydro-1H-1-benzazepin-3-yl)-3-(3-(1H-tetrazol-5-yl)phenyl)urea,
(-)-1-(1-diethylaminocarbonylmethyl-2-oxo-5-phenyl-2,3-dihydro-1H-1-benzazepin-3-yl)-3-(4-(1H-tetrazol-5-yl)phenyl)urea, and
(-)-1-(2-oxo-5-phenyl-1-(pyrrolidin-1-ylcarbonylmethyl)-2,3-dihydro-1H-1-benzazepin-3-yl)-3-(3-(1H-tetrazol-5-yl)phenyl)urea.
These compounds have particularly strong affinity for CCK-B receptor.

The pharmaceutically acceptable salts of the compound of the formula (I) include addition salts with inorganic acid or organic acid, and salts with inorganic base, organic base or amino acid, which are preferably substantially nontoxic in view of the object of the present invention.

The compounds of the present invention can be prepared, for example, by the following method.

Method 1

A compound which can be produced according to the method described in J. Med. Chem., vol. 14, p 40 (1971) and is represented by the formula

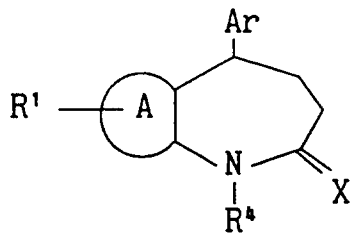

(II)

wherein each symbol is as defined above, is converted to a compound (III) by a method known in the field, which comprises introducing the compound (II) into a 3-halogen derivative (e.g., 3-chloro compound) by a treatment with phosphorus pentachloride and then hydrogenation, subjecting the obtained derivative to a treatment with phthalimide salt (e.g., potassium phthalimide), metal azide and the like, and reduction and hydrolysis as necessary, whereby a 3-aminoazepine compound of the formula

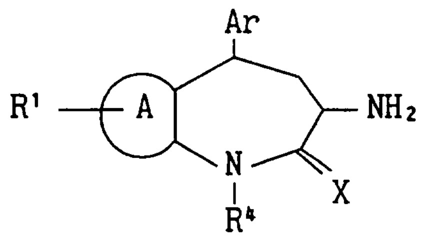

(III)

wherein each symbol is as defined above, can be obtained.

Method 2

The compound of the formula (III) is protected with a suitable amino protecting group such as phthaloyl, benzyloxycarbonyl, tert-butyloxycarbonyl and triphenylmethyl) to give a compound of the formula

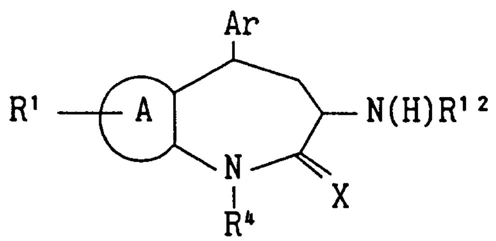

(IV)

wherein $R^{12}$ is a conventional protecting group of amino group, such as phthaloyl, benzyloxycarbonyl, tert-butoxycarbonyl and triphenylmethyl, and other symbols are as defined above.

Method 3

A compound of the formula

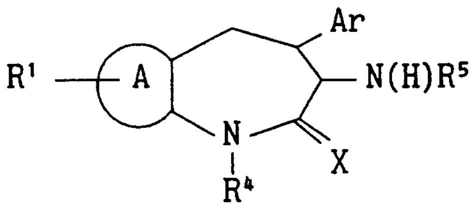

(V)

wherein each symbol is as defined above, can be obtained according to the method described in Japanese Patent Unexamined Publication No. 250354/1989).

Method 4

The compound of the formula (IV) or (V) is treated with a suitable halogenating agent, for example, by treating with halogen such as chlorine and bromine, or a halogenating agent such as NBS (N-bromosuccinimide) and NCS (N-chlorosuccinimide), in an inert solvent such as carbon tetrachloride. Where necessary, the treatment can be conducted in the presence of AIBN (azobisisobutyronitrile) or under irradiation of light. The obtained compound is subsequently treated under the basic conditions as necessary, for example, by treating in an inert solvent such as toluene and tetrahydrofuran using an organic base such as DBU (1,8-diazabicyclo[5.4.0]undeca-7-ene), potassium tert-butoxide, sodium methylate and sodium ethylate, at room temperature or under heating, or by the use of an aqueous solution of alkali such as sodium hydroxide and potassium hydroxide. Alternatively, the compound is heated. Then, the obtained compound is subjected to the elimination of the protecting group under suitable conditions to give a compound of the formula

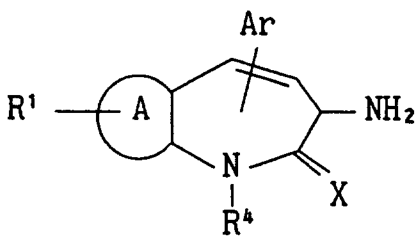

(VI)

wherein each symbol is as defined above.

The compound of the present invention can be also obtained by treating the compound of the formula (III), (IV), (V) or (VI) by one or two from the following methods in combination.

Method 5

The compound is reacted with a compound of the formula

$$R^{13}\text{-}Z \qquad (VII)$$

wherein $R^{13}$ is alkyl, aralkyl, a group of the formula

$$-(CH_2)_{l'}C(=Y)-R^6$$

wherein l' is an integer of 1-6 and other symbols are as defined above, or a group of the formula

$$-(CH_2)_{m'}S(O)_n-R^8$$

wherein m' is an integer of 1-6 and other symbols are as defined above, and Z is a group capable of leaving, such as halogen, methanesulfonyloxy and toluenesulfonyloxy.

The reaction proceeds in an inert solvent in the presence of a tertiary base such as triethylamine, pyridine and N,N-dimethylaniline, or an alkali such as sodium hydrogencarbonate, sodium carbonate, potassium carbonate, sodium hydroxide, potassium hydroxide, sodium hydride, sodium methoxide, sodium ethoxide, potassium tert-butoxide and lithium diisopropylamide, under cooling, at room temperature or under heating. Examples of the inert solvent include benzene, toluene, xylene, methanol, ethanol, ethyl ether, dioxane, tetrahydrofuran, chloroform, dichloromethane, dichloroethane, hexamethylphosphoric amide, diethylene glycol, dimethylformamide and mixed solvent thereof.

Method 6

The compound is reacted with an iso(thio)cyanate of the formula

$$R^7\text{-NCY} \qquad \text{(VIII)}$$

wherein each symbol is as defined above.

The reaction proceeds in a suitable solvent which does not inhibit the instant reaction. Examples of the solvent include organic solvents such as tetrahydrofuran, diethyl ether, diisopropyl ether, dioxane, dichloromethane, chloroform, ethyl acetate, benzene, toluene, xylene, dimethylformamide and dimethylacetamide. While the temperature of condensation varies depending on the reagent and solvent to be used, it is generally preferred to be between -20°C and the boiling point of the solvent.

Method 7

The compound is reacted with a carboxylic acid of the formula

$$R^6\text{-COOH} \qquad \text{(IX)}$$

wherein $R^6$ is as defined above, or a reactive derivative thereof. The reaction substantially belongs to amidation and the following amidation method and peptide synthesis known *per se* can be applied.

(1) When the compound of the formula (IX) is a free carboxylic acid, the reaction is carried out in an inert solvent in the presence of a condensing agent such as dicyclohexylcarbodiimide, titanium tetrachloride, phosphorus halide (e.g., phosphorus chloride and phosphorus oxychloride), diethyl chlorophosphite, o-phenylene chlorophosphite and ethyl dichlorophosphite, under cooling, at room temperature or under heating. The compound of the formula (III), (IV), (V) or (VI) may be previously reacted with a halogenated phosphorus in an inert solvent and condensed with the compound of the formula (IX). For example, when the halogenated phosphorus is phosphorus trichloride, about 1/2 mol of phosphorus trichloride is reacted with the compound of the formula (III), (IV), (V) or (VI), in an inert solvent in the presence of a tertiary base such as triethylamine, pyridine and N,N-dimethylaniline, under cooling or at room temperature, and then the obtained compound is reacted with the compound of the formula (IX) at room temperature or under heating, preferably under heating with reflux.
(2) When an acid halide (e.g., acid chloride and acid bromide) is used as a reactive derivative of the compound of the formula (IX), the reaction is carried out in an inert solvent in the presence of a tertiary base such as triethylamine, pyridine and N,N-dimethylaniline, under cooling or at room temperature, or in the presence of an alkali such as sodium hydroxide and potassium hydroxide, under cooling in water or at room temperature.
(3) When an acid azide is used as a reactive derivative of the compound of the formula (IX), the reaction is carried out in the presence of an alkali such as sodium hydroxide and potassium hydroxide, under cooling in water or at room temperature.
(4) When an ester such as methyl ester, ethyl ester, p-nitrophenyl ester and p-chlorophenyl ester is used as a reactive derivative of the compound of the formula (IX), the reaction is carried out in an inert solvent (or excess amount of the compound of the formula (III), (IV), (V) or (VI) used may act as a solvent) at room temperature or under heating, preferably under heating with reflux.
(5) When an asymmetric acid anhydride or a mixed acid anhydride such as alkyl-carbonic acid mixed acid anhydride, alkyl-phosphoric acid mixed acid anhydride, alkyl-phosphorous acid mixed acid anhydride and sulfuric acid mixed acid anhydride is used as a reactive derivative of the compound of the formula (IX), the reaction is carried out in an inert solvent in the presence of a tertiary base such as triethylamine, pyridine and N,N-dimethylaniline, under cooling, at room temperature or under heating.
(6) When an active amide such as acid imidazolide, acid pyrrolidide and 2,4-dimethylpyrazolide is used as a reactive derivative of the compound of the formula (IX), the reaction is carried out in an inert solvent at room temperature or under heating.

Examples of the inert solvent to be used in the aforementioned respective condensation reactions include benzene, toluene, xylene, methanol, ethanol, ethyl ether, dioxane, tetrahydrofuran, chloroform, dichloromethane, dichloroethane, hexamethylphosphoric amide, diethylene glycol, dimethylformamide and mixed solvent thereof, which is appropriately selected according to the kind of the reactive derivative.

Method 8

A compound of the formula (I) wherein $R^4$ and X are bonded to each other to form a group of the formula $-C(R^{10})=N-N=$ is obtained by reacting a compound of the formula (I) wherein X is oxygen and $R^4$ is hydrogen, namely, the compound of the formula

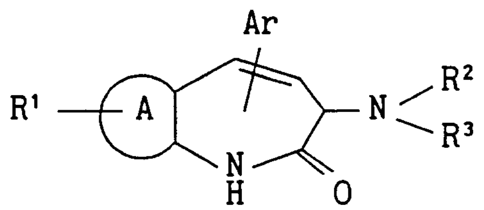

(X)

wherein each symbol is as defined above, with a thionating reagent to give a compound of the formula

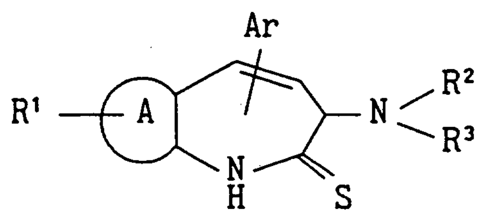

(XI)

wherein each symbol is as defined above, and reacting this compound of the formula (XI) with a compound of the formula

$$R^{10}CONHNH_2 \qquad (XII)$$

wherein $R^{10}$ is as defined above; or by reacting a compound of the formula (XI) with a hydrazine hydrate to give a compound of the formula

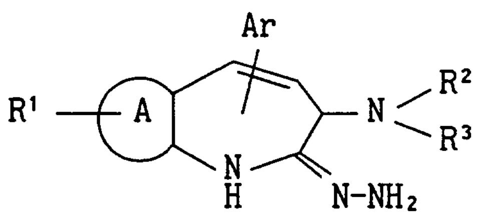

(XIII)

wherein each symbol is as defined above, and reacting this compound with a compound of the formula

$$R^{10}COOH \qquad (XIV)$$

wherein $R^{10}$ is as defined above, or a reactive derivative thereof, or a compound of the formula

$$R^{10}C(OR^{14})_3 \qquad (XV)$$

wherein $R^{14}$ is alkyl such as methyl and ethyl and $R^{10}$ is as defined above.

In the above-mentioned methods, examples of the thionating reagent include phosphorus pentasulfide and Lawesson's reagent [2,4-bis(4-methoxyphenyl)-1,3,2,4-dithiadiphosphetane-2,4-disulfide], and examples of the reactive derivative of the compound of the formula (XIV) include acid halide, acid anhydride, mixed acid anhydride, $C_1$-$C_5$ alkyl ester and benzyl ester.

The reaction between the compound of the formula (X) and the thionating reagent generally proceeds in a solvent inert to the reaction, such as benzene, toluene, xylene, tetrahydrofuran, chloroform, dioxane and mixed solvent thereof, at 30-100°C for 30 minutes to 5 hours.

The reaction between the compound of the formula (XI) and the compound of the formula (XII) generally proceeds in a solvent inert to the reaction, such as benzene, toluene, xylene, tetrahydrofuran, dioxane and mixed solvent thereof, in the presence of an organic acid such as acetic acid and propionic acid, inorganic acid such as hydrochloric acid and sulfuric acid or silica gel, at room temperature to the refluxing temperature of the solvent used, for 30 minutes to 5 hours. The reaction between the compound of the formula (XI) and the hydrazine hydrate generally proceeds in a solvent inert to the reaction, such as methanol, ethanol, propanol, isopropyl alcohol and butanol, at 0-40°C for 5 minutes to about 3 hours.

The reaction between the compound of the formula (XIII) and the compound of the formula (XIV) or a reactive derivative thereof or a compound of the formula (XV) proceeds in a solvent inert to the reaction, such as benzene, toluene, xylene, tetrahydrofuran, dioxane and mixed solvent thereof, in the presence of an organic acid such as acetic acid and propionic acid, inorganic acid such as hydrochloric acid and sulfuric acid or silica gel, at room temperature to the refluxing temperature of the solvent used, for 30 minutes to 6 hours.

<u>Method 9</u>

A more preferable production method includes the following steps. A compound of the formula

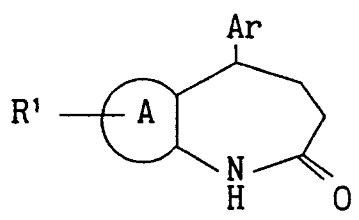

(XVI)

wherein each symbol is as defined above, which can be obtained according to the process described in J. Med. Chem., vol. 14, p 40 (1971), is converted to a 3-halogen derivative according to the process of Method 1, and reacted with a phthalimide salt (e.g., potassium phthalimide) in a solvent inert to the reaction, such as dimethylformamide, toluene, xylene, benzene and mixed solvent thereof, at room temperature to the refluxing temperature of the solvent used, for 1 to 24 hours to give a compound of the formula

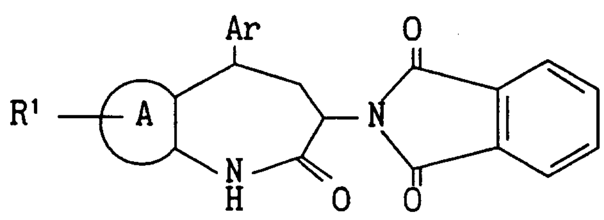

(XVII)

wherein each symbol is as defined above. The compound of the formula (XVII) is reacted with a compound of the formula

$R^4$-Z (XVIII)

wherein each symbol is as defined above, to give a compound of the formula

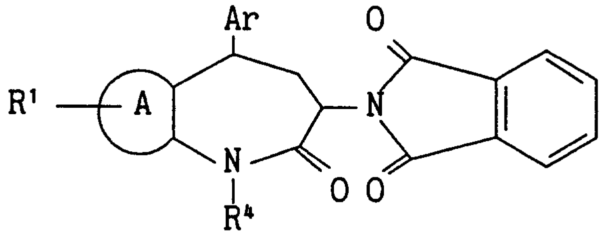

(XIX)

wherein each symbol is as defined above, and this compound is treated according to the process of Method 4, and then according to one or two from Method 5, Method 6 and Method 7 to give a compound of the formula (I).

The reaction between the compound of the formula (XVII) and the compound of the formula (XVIII) is carried out in an inert solvent in the presence of a tertiary base such as triethylamine, pyridine and N,N-dimethylaniline, or an alkali such as sodium hydrogencarbonate, sodium carbonate, potassium carbonate, sodium hydroxide, potassium hydroxide, sodium hydride, sodium methoxide, sodium ethoxide, potassium tert-butoxide and lithium diisopropylamide, under cooling, at room temperature or under heating. Examples of the inert solvent include benzene, toluene, xylene, methanol, ethanol, ethyl ether, dioxane, tetrahydrofuran, chloroform, dichloromethane, dichloroethane, hexamethylphosphoric amide, diethylene glycol, dimethylformamide and mixed solvent thereof.

Method 10

The compound of the formula (I) wherein $R^4$ is represented by the formula

$$-(CH_2)_pCOR^9$$

wherein p is 0 or an integer of 1-6 and $R^9$ is hydroxy, namely, the compound of the formula

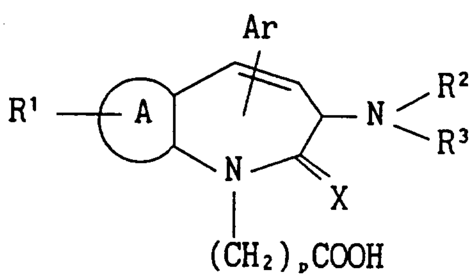

(XX)

wherein each symbol is as defined above, can be easily produced by subjecting the compound of the formula (I) wherein $R^4$ is represented by the formula

$$-(CH_2)_pCOR^9$$

wherein p is 0 or an integer of 1-6 and $R^9$ is alkoxy, to hydrolysis known in the pertinent field.

Method 11

The compound of the formula (I) wherein $R^4$ is represented by the formula

$$-(CH_2)_pCOR^9$$

wherein p is 0 or an integer of 1-6 and $R^9$ is amino, alkylamino, arylamino, aralkylamino, or a cyclic amino of the formula (a) can be produced by condensing the compound of the formula (XX) with an amine of the formula

$$R^9\text{-}H \qquad (XXI)$$

wherein $R^9$ is as defined above, or a reactive derivative thereof. The reaction substantially belongs to amidation and the following amidation method and peptide synthesis known *per se* can be applied.

(1) When the compound of the formula (XX) is a free carboxylic acid, the reaction is carried out in an inert solvent in the presence of a condensing agent such as dicyclohexylcarbodiimide, titanium tetrachloride, phosphorus halide (e.g., phosphorus chloride and phosphorus oxychloride), diethyl chlorophosphite, o-phenylene chlorophosphite and ethyl dichlorophosphite, under cooling, at room temperature or under heating.
(2) When an acid halide (e.g., acid chloride and acid bromide) is used as a reactive derivative of the compound of the formula (XX), the reaction is carried out in an inert solvent in the presence of a tertiary base such as triethylamine, pyridine and N,N-dimethylaniline, under cooling or at room temperature, or in the presence of an alkali such as sodium hydroxide and potassium hydroxide under cooling in water or at room temperature.
(3) When an acid azide is used as a reactive derivative of the compound of the formula (XX), the reaction is carried out in the presence of an alkali such as sodium hydroxide and potassium hydroxide under cooling in water or at room temperature.
(4) When an ester such as methyl ester, ethyl ester, p-nitrophenyl ester and p-chlorophenyl ester is used as a reactive derivative of the compound of the formula (XX), the reaction is carried out in an inert solvent, at room temperature or under heating, preferably under heating with reflux.
(5) When an asymmetric acid anhydride or a mixed acid anhydride such as alkyl-carbonic acid mixed acid anhydride, alkyl-phosphoric acid mixed acid anhydride, alkyl-phosphorous acid mixed acid anhydride and sulfuric acid mixed acid anhydride is used as a reactive derivative of the compound of the formula (XX), the reaction is carried out in an inert solvent in the presence of a tertiary base such as triethylamine, pyridine and N,N-dimethylaniline, under cooling or under heating.
(6) When an active amide such as acid imidazolide, acid pyrrolidide and 2,4-dimethylpyrazolide is used as a reactive derivative of the compound of the formula (XX), the reaction is carried out in an inert solvent at room temperature or under heating.

Examples of the inert solvent to be used in the aforementioned respective condensation reactions include benzene, toluene, xylene, methanol, ethanol, ethyl ether, dioxane, tetrahydrofuran, chloroform, dichloromethane, dichloroethane, hexamethylphosphoric amide, diethylene glycol, dimethylformamide and mixed solvent thereof, which is appropriately selected according to the kind of the reactive derivative.

The compound of the formula (I) thus obtained can be separated and purified from the reaction mixture by a method known *per se*, such as recrystallization and column chromatography.

The compound of the formula (I) can be converted to a salt by treating with an inorganic acid such as hydrochloric acid, hydrobromic acid, sulfuric acid, phosphoric acid and nitric acid, organic acid such as acetic acid, propionic acid, succinic acid, glycolic acid, lactic acid, malic acid, tartaric acid, citric acid, ascorbic acid, maleic acid, fumaric acid, methanesulfonic acid, toluenesulfonic acid and benzenesulfonic acid, inorganic base such as sodium hydroxide, potassium hydroxide, calcium hydroxide, magnesium hydroxide, zinc hydroxide and ammonium hydroxide, organic base such as methylamine, diethylamine, triethylamine, dicyclohexylamine, triethanolamine, ethylenediamine, trishydroxymethylaminomethane, quinine, guanidine and cinchonine, or amino acid such as lysine, ornithine, arginine and alanine, according to a conventional method.

When the compound of the present invention has a chiral carbon atom, the compound is generally obtained as a racemate. The racemate can be resolved into optical isomers by a conventional method. It can be also resolved by liquid chromatography using an optically active column. Alternatively, it may be converted to a diastereomer by condensing with an optically active compound, isolated into an optically active derivative by fractional recrystallization or column chromatography using suitable solvent, and subjected to hydrolysis. Such optical isomers can be also produced by using an optically active starting material. Respective diastereomers can be purified by fractional crystallization or chromatography.

The pharmacological action of the compound (I) is determined as in the following.

<u>Experimental Example 1</u> : CCK-A receptor binding test (pancreas)

The whole pancreas was removed from a male rat. The fat tissues were removed and the pancreas was homogenized (Brinkman Polytron PT20) in 50 mM Tris hydrochloric acid (pH 7.5). The homogenate was filtered through a nylon cloth (120 mesh) and centrifuged at 50,000 × g for 12 minutes. The obtained sediment was homogenized in Tris buffer as in the above and centrifuged. The sediment was suspended in a buffer for binding assay [50 mM Tris-HCl (pH 7.2) containing 5 mM magnesium chloride, 5 mM dithiothreitol, 2 mg/ml bovine serum albumin, 0.1 mg/ml bacitracin and 0.14 mg/ml trypsin inhibitor (soybean)] and used as a receptor source.

For binding assay, 50 μl of a buffer (for total binding), unlabeled CCK-8 sulfate (for nonspecific binding) having a final concentration of 1 5M or a test compound (for determining inhibition of $^{125}$I-CCK binding) and 50 μl of $^{125}$I-CCK-8

(63-67 TBq/mmol, 40,000-50,000 cpm) were added to a membrane suspension (450 µl, containing 100 µg protein). The reaction mixture was incubated at 20°C for 30 minutes, filtered by suction through a glass fiber filter (Whatman GF/B), and immediately thereafter washed 3 times with ice-cooled Tris buffer (2.5 ml). The radioactive concentration on the filter was measured.

Experimental Example 2 : CCK-B receptor binding test (brain)

In the case of the central nervous system, cerebral cortex from rat was prepared and suspended in a buffer, pH 6.5, containing 5 mM magnesium chloride, 1 mM EGTA [ethylene glycol bis(2-aminoethylether)tetraacetic acid], 360 mM sodium chloride, 15 mM potassium chloride, 20 mM HEPES (N-2-hydroxyethylpiperazine-N'-2-ethanesulfonic acid) and 0.25 mg/ml bacitracin. For binding assay, 50 µl of a buffer (for total binding), unlabeled CCK-8 sulfate (for nonspecific binding) having a final concentration of 1 µM or a test compound (for determining inhibition of $^{125}$I-CCK binding) and 50 µl of $^{125}$I-CCK-8 (63-67 TBq/mmol, 40,000 - 50,000 cpm) were added to a membrane suspension (450 µl, containing 100 µg protein). The reaction mixture was incubated at 20°C for 30 minutes, filtered by suction through a glass fiber filter (Whatman GF/B), and immediately thereafter washed 3 times with ice-cooled Tris buffer (2.5 ml). The radioactive concentration on the filter was measured.

The effects of the test compound with regard to the CCK receptor binding in Experimental Examples 1 and 2 were evaluated by determining an inhibition ratio by the following formula, which shows the concentration ($IC_{50}$, nM) inhibiting 50% of the specific binding. The results are shown in Table 1.

$$\text{Inhibition (\%)} = 100 - \frac{\text{(binding when compound added - nonspecific binding)}}{\text{(total binding - nonspecific binding)}} \times 100$$

Table 1

| test compounds | binding affinity (Ki,nM) | | selectivity (A/B) |
|---|---|---|---|
| | CCK-A | CCK-B | |
| Example 36 | 4.7 | >1000 | <0.0047 |
| Example 46 | 100 | 0.87 | 110 |
| Example 48 | 210 | 2.1 | 100 |
| Example 52 | 110 | 0.92 | 120 |
| Example 66 | 310 | 2.5 | 124 |
| Example 64 | 200 | 1.1 | 180 |
| Example 68 | 150 | 1.1 | 140 |
| Example 74 | 290 | 2.4 | 120 |
| Example 81 | 42 | >1000 | <0.042 |
| L-365260 | 390 | 19 | 21 |

The comparison compound L-365260 is (R)-N-(2,3-dihydro-1-methyl-2-oxo-5-phenyl-1H-1,4-benzodiazepin-3-yl)-N'-(3-methylphenyl)urea which is disclosed in US Patent No. 4,820,834.

As is evident from the results shown in Table 1, the compounds of Example 36 and Example 81 showed strong, selective affinity for CCK-A receptor and the other compounds showed strong, selective affinity for CCK-B receptor.

Experimental Example 3 : gastrin receptor binding test

Gastrin mucosal membrane obtained from guinea pig was immersed in a buffer, pH 7.4, containing 130 mM sodium chloride, 12 mM sodium bicarbonate, 3 mM sodium dihydrogenphosphate, 3 mM disodium hydrogenphosphate, 3 mM dipotassium hydrogenphosphate, 2 mM magnesium sulfate, 1 mM calcium chloride, 5 mM glucose, 4 mM L-glutamine and 25 mM HEPES, and incubated at 37°C for 40 minutes. The gastrin gland was released from this tissue and filtered through 200 mesh nylon. The filtered gastrin gland was centrifuged at 270 g for 5 minutes and washed twice by resuspension and centrifugation. For binding assay, 50 µl of a buffer (for total binding), unlabeled CCK-8 sulfate (for nonspecific binding) having a final concentration of 1 µM or a test compound (for determining inhibition of $^{125}$I-CCK binding) and 50

μl of $^{125}$I-CCK-8 (63-67 TBq/mmol, 40,000 - 50,000 cpm) were added to a membrane suspension (450 μl, containing 100 μg protein). The reaction mixture was incubated at 20°C for 30 minutes, filtered by suction through a glass fiber filter (Whatman G/FB), and immediately thereafter washed 3 times with ice-cooled Tris buffer (2.5 ml). The radioactive concentration on the filter was measured.

Experimental Example 4 : *ex vivo* CCK receptor binding test

The test compound was administered to the test animals and the animals were killed by decapitation after a predetermined time. The brain was removed and the membrane specimens were prepared, and the receptor binding performance was measured according to Experimental Example 2. Inhibition of *ex vivo* CCK receptor binding performance was calculated based on the level of specific binding in the group administered with solvent as 100%.

Experimental Example 5 : rat social interaction test (antianxiety effect)

Male Lister hooded rats were used as 5 pairs per group. The test included administration of the test compound, placing a pair (two rats) of rats from different home cages in a test apparatus under 1200 lux illumination, videotaping their behavior for 10 minutes and analyzing the behavior. The indices of the behavior analyzed were the total time (SI time) of smelling and tail chasing between the rats which are called social interaction, and the number of times the rats crossed the line drawn on the apparatus floor. The former was used as the index of the anxiety condition of the animals and the latter that of locomotor activity.

Experimental Example 6 : rat water-lick conflict test (antianxiety effect)

Wistar rats deprived of water for 48 hours were used. After the administration of the test compound, the rats were placed in the test apparatus, and the number of electric shock applied along with the water-drinking behavior for 3 minutes was recorded.

Experimental Example 7 : calculation of bioavailability (BA)

SD male and female rats weighing 200-300 g (supplied by Seiwa Test Animals Corp.) were used. The test compound was administered intravenously or orally and blood samples were taken with the lapse of time from orbital vein layer while the animals were under light anesthetization with ether. The drug concentration in plasma was measured by the HPLC method. The BA value (F value) after the oral administration was calculated from the following formula.

$$F = \frac{AUC_{\text{o-th, po}} \text{ x } Dose_{\text{iv}}}{AUC_{\text{o-th, iv}} \text{ x } Dose_{\text{po}}} \times 100$$

Experimental Example 8 : calculation of intracerebral transition (Kp)

SD male rats weighing 200-300 g (supplied by Seiwa Test Animals Corp.) were used. The rats were fixed on the dorsal position under anesthetization with urethane, cannulated at the left femoral vein and intravenously administered at a constant rate. When the concentration in plasma reached a stationary stage, the rats were decapitated and the brain and blood were respectively taken. The drug concentration in brain and plasma was measured by the HPLC method. The Kp value at the stationary stage was calculated from the following formula.

$$Kp_{ss} = \frac{C_b}{C_p} \times 100$$

wherein $C_b$ and $C_p$ respectively mean concentration in brain and concentration in plasma.

Experimental Example 9 : acute toxicity

Male ddY mice were used at 10 per group. The compounds of Example 36 and Example 46 were intraperitoneally administered by 300 mg/kg and the mice were monitored for 5 days. No death case was found with regard to the both compounds.

As described supra, the compound of the present invention showed strong affinity for CCK receptors and is useful as a highly safe pharmaceutical agent. Of the compounds of the present invention, those having 2-indolcarboxamide at the 3-position showed particularly strong affinity for CCK-A receptor and those having ureido at the 3-position showed particularly strong affinity for CCK-B receptor. The compounds of the present invention characteristically have superior dissolution property, high intracerebral transition, extremely high bioavailability and superior duration of effects.

The compound of the present invention and acid addition salts thereof can be used as a pharmaceutical composition as they are or on admixing with carriers and excipients known *per se*, such as lactose, starch, sucrose and magnesium stearate. The administration route may be either oral or parenteral. The composition for oral administration may be solid or liquid. Typical dosage form includes, for example, tablet, pill, granule, powder, capsule, syrup, emulsion and suspension. The composition for parenteral administration includes, for example, injection, suppository, inhalant and percutaneous absorber, and examples of injection include subcutaneous injection, intradermic injection and intramuscular injection. Such injections can be prepared by a method known *per se*, that is, by suspending or emulsifying these compounds in a sterile aqueous solution (e.g., physiological saline and isotonic solution) or oily liquid (e.g., sesame oil and soybean oil) conventionally used for injections. Where necessary, a suitable suspending agent (e.g., sodium carboxymethylcellulose and nonionic surfactant), solubilizer (e.g., benzyl benzoate and benzyl alcohol) and the like may be also used. While the dose varies depending on administration target, administration route, symptom and the like, it is generally 0.1-500 mg, preferably 0.1-100 mg daily for an adult.

## Best Mode for Embodying the Invention

The present invention is described in detail by way of Examples. It is needless to say that the present invention is not limited to these Examples. In the Examples to follow, the melting point noted was measured in the capillary, and nuclear magnetic resonance (NMR) was recorded using tetramethylsilan as an internal standard.

### Example 1

5-Phenyl-2,3,4,5-tetrahydro-1H-1-benzazepin-2-one (2.0 g) synthesized according to J. Med. Chem., vol. 14, p 40 (1971) and phosphorus pentachloride (5.3 g) in xylene (35 ml) are heated to 75°C, and the mixture is stirred for 3 hours. The solvent is distilled away, water (10 ml) is added, and the mixture is extracted with ethyl acetate (100 ml). After drying over anhydrous magnesium sulfate, the residue is concentrated under reduced pressure and purified by silica gel column chromatography (developing solvent: hexane : ethyl acetate = 5 : 1) to give 3,3-dichloro-5-phenyl-2,3,4,5-tetrahydro-1H-1-benzazepin-2-one (0.85 g), melting point 222-224°C (dec.).

### Example 2

3,3-Dichloro-5-phenyl-2,3,4,5-tetrahydro-1H-1-benzazepin-2-one (0.85 g), sodium acetate (0.57 g), sodium hydrophosphite (0.32 g) and 10% palladium-carbon (0.10 g) in acetic acid (50 ml) are stirred for 3 hours. The reaction mixture is filtered and the filtrate is concentrated, added with water (10 ml) and the mixture is extracted with ethyl acetate (100 ml). The extract is washed with aqueous sodium hydrogencarbonate solution, dried over anhydrous magnesium sulfate and concentrated under reduced pressure. Purification by silica gel column chromatography (developing solvent: hexane : ethyl acetate = 2 : 1) gave 3-chloro-5-phenyl-2,3,4,5-tetrahydro-1H-1-benzazepin-2-one (0.11 g), melting point 162-163°C (dec.).

### Example 3

3-Chloro-5-phenyl-2,3,4,5-tetrahydro-1H-1-benzazepin-2-one (0.14 g) and potassium phthalimide (0.11 g) in dimethylformamide (3 ml) is heated to 50°C and stirred for 24 hours. Water (5 ml) is added and the mixture is extracted with ethyl acetate (50 ml). After drying over anhydrous magnesium sulfate, the residue is concentrated under reduced pressure. The resulting crystals are collected by filtration to give 3-phthaloylamino-5-phenyl-2,3,4,5-tetrahydro-1H-1-benzazepin-2-one (0.03 g), melting point 237°C (dec.).

### Example 4

3-Phthaloylamino-5-phenyl-2,3,4,5-tetrahydro-1H-1-benzazepin-2-one (4.4 g), sodium hydride (60% oil mixture, 0.55 g) and methyl iodide (1.72 ml) in dimethylformamide (50 ml) are stirred under ice-cooling for one hour. The reaction mixture is poured in ice water and extracted with ethyl acetate (100 ml). After drying over anhydrous magnesium sulfate, the residue is concentrated under reduced pressure and crystallized from a mixed solvent of ethyl acetate - hexane. The crystals are collected by filtration to give 1-methyl-3-phthaloylamino-5-phenyl-2,3,4,5-tetrahydro-1H-1-benzazepin-2-one (2.4 g), melting point 205-209°C.

### Example 5

1-Methyl-3-phthaloylamino-5-phenyl-2,3,4,5-tetrahydro-1H-1-benzazepin-2-one (0.37 g), N-bromosuccinimide (0.38 g) and azobisisobutyronitrile (0.01 g) in carbon tetrachloride (25 ml) are stirred for one hour under irradiation of

white light. The reaction mixture is filtered and the filtrate is concentrated. Water (10 ml) is added and the mixture is extracted with chloroform (100 ml). After drying over anhydrous magnesium sulfate, the residue is concentrated under reduced pressure. Purification by silica gel column chromatography (developing solvent: hexane : ethyl acetate = 2 : 1) gave 1-methyl-3-phthaloylamino-5-phenyl-2,3-dihydro-1H-1-benzazepin-2-one (0.37 g).
$^1$H-NMR($CDCl_3$); 2.39(3/2H,s), 3.48(3/2H,s), 4.97(1/2H,d), 5.17(1/2H,d), 6.83(1/2H,d), 7.00(1/2H,d)

Example 6

1-Methyl-3-phthaloylamino-5-phenyl-2,3-dihydro-1H-1-benzazepin-2-one (0.37 g) and hydrazine·monohydrate (0.23 ml) in methanol (40 ml) are refluxed for one hour. Water (10 ml) is added, methanol is distilled away under reduced pressure and the residue is extracted with chloroform (100 ml). After drying over anhydrous magnesium sulfate, the residue is concentrated under reduced pressure. Purification by silica gel column chromatography (developing solvent: chloroform : methanol = 20 : 1) gave 3-amino-1-methyl-5-phenyl-2,3-dihydro-1H-1-benzazepin-2-one (0.11 g).
$^1$H-NMR($CDCl_3$); 1.94(2H,s), 3.48(3H,s), 3.63(1H,d), 5.94(1H,d)

Example 7

3-Amino-1-methyl-5-phenyl-2,3-dihydro-1H-1-benzazepin-2-one (0.11 g) and m-tolylisocyanate (0.05 ml) in benzene (5 ml) are stirred for one hour. The resulting crystals are collected by filtration to give 0.12 g of crystals. The crystals are recrystallized from ethyl acetate to give 1-(1-methyl-2-oxo-2,3-dihydro-1H-1-benzazepin-3-yl)-3-(3-methylphenyl)urea, melting point 226-227°C.

Example 8

3-Amino-4-phenyl-2,3,4,5-tetrahydro-1H-1-benzazepin-2-one (3.0 g) which can be prepared by the method described in Japanese Patent Unexamined Publication No. 25354/1989 is dissolved in a mixed solvent of dioxane - water (2 : 1). 1N Aqueous sodium hydroxide solution (40 ml) and di-tert-butyl dicarbonate (3 g) are added, and the mixture is stirred for 2 hours. 10% Aqueous citric acid solution is added to the reaction mixture to adjust same to pH 3 and the mixture is extracted with ethyl acetate (500 ml). After drying over anhydrous magnesium sulfate, the residue is concentrated under reduced pressure. The concentrated residue is dissolved in dimethylformamide (50 ml), and sodium hydride (60% oil mixture, 0.48 g) is added. The mixture is stirred at room temperature for 0.5 hour. Methyl iodide (1.0 ml) is added and the mixture is stirred for 7 hours. The reaction mixture is concentrated, added with water (10 ml) and extracted with ethyl acetate (100 ml). After drying over anhydrous magnesium sulfate, the residue is concentrated under reduced pressure, and a mixed solvent of diisopropyl ether - hexane is added to the residue for crystallization. The crystals are collected by filtration to give 3-tert-butoxycarbonylamino-1-methyl-4-phenyl-2,3,4,5-tetrahydro-1H-1-benzazepin-2-one (3.2 g), melting point 195-197°C.

Example 9

3-tert-Butoxycarbonylamino-1-methyl-4-phenyl-2,3,4,5-tetrahydro-1H-1-benzazepin-2-one (2.0 g) is mixed with carbon tetrachloride (100 ml), and N-bromosuccinimide (1.1 g) and azobisisobutyronitrile (0.03 g) are added. The mixture is stirred for 1.5 hours under irradiation of white light. The reaction mixture is filtered and the filtrate is concentrated. Water (10 ml) is added and the mixture is extracted with chloroform (100 ml). After drying over anhydrous magnesium sulfate, the residue is concentrated under reduced pressure and purified by silica gel column chromatography (developing solvent: hexane : ethyl acetate = 4 : 1) to give 4-bromo-3-tert-butoxycarbonylamino-1-methyl-4-phenyl-2,3,4,5-tetrahydro-1H-1-benzazepin-2-one (0.22 g) [$^1$H-NMR($CDCl_3$); 1.36(9H,s), 3.50(3H,s), 3.63(1H,d,J=14Hz), 3.85(1H,d,J=14Hz), 4.96(1H,d,J=10Hz), 5.70(1H,d,J=10Hz), 6.57(1H), 6.98(1H), 7.2-7.4(5H), 7.50-7.53(2H)] and 5-bromo-3-tert-butoxycarbonylamino-1-methyl-4-phenyl-2,3,4,5-tetrahydro-1H-1-benzazepin-2-one (1.2 g) [$^1$H-NMR($CDCl_3$); 1.14(9H,s), 3.48(3H,s), 3.72(1H,d,J=12Hz), 4.71(1H,dd,J=9Hz,12Hz), 5.24(1H,d,J=9Hz)].

Example 10

4-Bromo-3-tert-butoxycarbonylamino-1-methyl-4-phenyl-2,3,4,5-tetrahydro-1H-1-benzazepin-2-one (200 mg) is dissolved in toluene (20 ml). 1,8-Diazabicyclo[5.4.0]undeca-7-ene (0.08 ml) is added and the mixture is refluxed for 2 hours. Water (5 ml) is added and the mixture is extracted with ethyl acetate (50 ml). After washing with aqueous citric acid solution and water, the residue is dried over anhydrous magnesium sulfate. The residue is concentrated under reduced pressure and purified by silica gel column chromatography (developing solvent: hexane : ethyl acetate = 4 : 1) to give 3-tert-butoxycarbonylamino-1-methyl-4-phenyl-2,3-dihydro-1H-1-benzazepin-2-one (100 mg).
$^1$H-NMR($CDCl_3$); 1.22(9H,s), 3.47(3H,s), 4.63(1H,dd,J=7Hz,2Hz), 5.90(1H,d,J=7Hz), 6.74(1H,d,J=2Hz), 7.2-7.4(9H)

Example 11

To 3-tert-butoxycarbonylamino-1-methyl-4-phenyl-2,3-dihydro-1H-1-benzazepin-2-one (50 mg) is added trifluoroacetic acid (1.0 ml) and the mixture is stood for 0.5 hour, followed by concentration under reduced pressure. Diisopropyl ether is added to the residue for crystallization. The crystals are collected by filtration to give 3-amino-1-methyl-4-phenyl-2,3-dihydro-1H-1-benzazepin-2-one. trifluoroacetate (50 mg), melting point 190-193°C.

Example 12

To a solution of 3-amino-1-methyl-4-phenyl-2,3-dihydro-1H-1-benzazepin-2-one · trifluoroacetate (40 mg), dichloromethane (1.0 ml) and triethylamine (0.03 ml) is added m-tolylisocyanate (0.02 ml), and the mixture is stirred at room temperature for 3 hours. Water (5 ml) is added and the mixture is extracted with chloroform (50 ml). After drying over anhydrous magnesium sulfate, the residue is concentrated under reduced pressure. Diisopropyl ether is added to the residue for crystallization. The crystals are recrystallized from ethyl acetate to give 1-(1-methyl-2-oxo-4-phenyl-2,3-dihydro-1H-1-benzazepin-3-yl)-3-(3-methylphenyl)urea (20 mg), melting point 247-248°C.

Example 13

5-Bromo-3-tert-butoxycarbonylamino-1-methyl-4-phenyl-2,3,4,5-tetrahydro-1H-1-benzazepin-2-one (450 mg) is dissolved in tetrahydrofuran (10 ml), and potassium tert-butoxide (150 mg) is added. The mixture is stirred for 3 hours. Water (10 ml) is added and the mixture is extracted with ethyl acetate (100 ml). After washing with aqueous citric acid solution and water, the residue is dried over anhydrous magnesium sulfate. The residue is concentrated under reduced pressure and purified by silica gel column chromatography (developing solvent: hexane : ethyl acetate = 3 : 1) to give 3-tert-butoxycarbonylamino-1-methyl-4-phenyl-2,3-dihydro-1H-1-benzazepin-2-one (290 mg).
$^1$H-NMR($CDCl_3$); 1.22(9H,s), 3.47(3H,s), 4.63(1H,dd,J=2Hz,7Hz), 5.90(1H,d,J=7Hz), 6.74(1H,d,J=2Hz), 7.2-7.4(9H)

Example 14

3-tert-Butoxycarbonylamino-1-ethoxycarbonylmethyl-4-phenyl-2,3,4,5-tetrahydro-1H-1-benzazepin-2-one (0.44 g) is mixed with carbon tetrachloride (10 ml). N-Bromosuccinimide (0.19 g) and azobisisobutyronitrile (0.01 g) are added, and the mixture is stirred for 0.5 hour under irradiation of white light. The reaction mixture is filtered and the filtrate is concentrated. Water (5 ml) is added and the mixture is extracted with chloroform (50 ml). After drying over anhydrous magnesium sulfate, the residue is concentrated under reduced pressure and the residue is purified by silica gel column chromatography (developing solvent: hexane : ethyl acetate = 4 : 1) to give 4-bromo-3-tert-butoxycarbonylamino-1-ethoxycarbonylmethyl-4-phenyl-2,3,4,5-tetrahydro-1H-1-benzazepin-2-one (240 mg) [$^1$H-NMR($CDCl_3$); 1.26(3H,t,J=7Hz), 1.36(9H,s), 3.63(1H,d,J=14Hz), 4.19(2H,q,J=7Hz), 4.34(1H,d,J=17Hz), 4.47(1H,d,J=14Hz), 5.01(1H,d,J=10Hz), 5.03(1H,d,J=17Hz), 5.69(1H,d,J=10Hz), 6.59(1H,d,J=7Hz), 7.00(1H,dd,J=1Hz,8Hz), 7.2-7.5(5H), 7.51(2H,dd,J=2Hz,7Hz)
$^1$H-NMR($CDCl_3$); 1.24(3H,t,J=7Hz), 1.35(9H,s), 3.39(1H,d,J=15Hz), 4.18(2H,q,J=7Hz), 4.26(1H,d,J=17Hz), 4.52(1H,d,J=15Hz), 4.77(1H,d,J=10Hz), 4.84(1H,d,J=10Hz), 5.06(1H,d,J=17Hz), 7.2-7.5(7H), 7.87(2H,d,J=7Hz), (note: mixture of diastereomers)] and 5-bromo-3-tert-butoxycarbonylamino-1-ethoxycarbonylmethyl-4-phenyl-2,3,4,5-tetrahydro-1H-1-benzazepin-2-one (140 mg) [$^1$H-NMR($CDCl_3$); 1.14(9H,s), 1.34(3H,t,J=7Hz), 3.69(1H,d,J=12Hz), 3.82(1H,d,J=17Hz), 4.31(2H,q,J=7Hz), 4.79(1H,dd,J=9Hz,12Hz), 5.12(1H,d,J=17Hz), 5.17(1H,s), 5.26(1H,d,J=9Hz), 7.1-7.5(9H)].

Example 15

4-Bromo-3-tert-butoxycarbonylamino-1-ethoxycarbonylmethyl-4-phenyl-2,3,4,5-tetrahydro-1H-1-benzazepin-2-one (140 mg) is dissolved in toluene (20 ml). 1,8-Diazabicyclo[5.4.0]undeca-7-ene (0.05 ml) is added and the mixture is refluxed for 2 hours. Water (5 ml) is added and the mixture is extracted with ethyl acetate (50 ml). After washing with aqueous citric acid solution and water, the residue is concentrated under reduced pressure and the residue is purified by silica gel column chromatography (developing solvent: hexane : ethyl acetate = 4 : 1) to give 3-tert-butoxycarbonylamino-1-ethoxycarbonylmethyl-4-phenyl-2,3-dihydro-1H-1-benzazepin-2-one (100 mg).
$^1$H-NMR($CDCl_3$); 1.23(9H,s), 1.24(3H,t,J=7Hz), 4.21(2H,q,J=7Hz), 4.50(1H,d,J=17Hz), 4.63(1H,d,J=17Hz), 4.75(1H,dd,J=2Hz,7Hz), 5.83(1H,d,J=7Hz), 6.78(1H,d,J=2Hz), 7.2-7.4(9H)

Example 16

To 3-tert-butoxycarbonylamino-1-ethoxycarbonylmethyl-4-phenyl-2,3-dihydro-1H-1-benzazepin-2-one (120 mg) is added trifluoroacetic acid (2.0 ml). The mixture is stood for 0.5 hour and concentrated under reduced pressure. Diisopropyl ether is added to the residue for crystallization. The crystals are collected by filtration to give 3-amino-1-ethoxycarbonylmethyl-4-phenyl-2,3-dihydro-1H-1-benzazepin-2-one trifluoroacetate (120 mg), melting point 190-193°C.

Example 17

To a solution of 3-amino-1-ethoxycarbonylmethyl-4-phenyl-2,3-dihydro-1H-1-benzazepin-2-one trifluoroacetate (120 mg), dichloromethane (1.0 ml) and triethylamine (0.11 ml) is added m-tolylisocyanate (0.06 ml), and the mixture is stirred at room temperature for 11 hours. Ethyl acetate is added to the reaction mixture for crystallization. The crystals are collected by filtration to give 1-(1-ethoxycarbonylmethyl-2-oxo-4-phenyl-2,3-dihydro-1H-1-benzazepin-3-yl)-3-(3-methylphenyl)urea (100 mg), melting point 234-236°C.

Example 18

3-Amino-1-methyl-5-phenyl-2,3-dihydro-1H-1-benzazepin-2-one (37.7 g) is dissolved in isopropyl alcohol (250 ml). D-(+)-Dibenzoyltartaric acid (20.1 g) is dissolved in water (250 ml). The both mixtures are mixed under heating. The mixture is stood at room temperature for one day and the resulting crystals are collected by filtration to give 3-amino-1-methyl-5-phenyl-2,3-dihydro-1H-1-benzazepin-2-one. D-(+)-dibenzoyltartrate (9.0 g), melting point 166-169°C (dec.). The filtrate is concentrated under reduced pressure, added with 5% aqueous potassium carbonate solution to make same alkaline and extracted with chloroform (500 ml). After washing with water, the residue is dried over anhydrous magnesium sulfate and concentrated under reduced pressure. The concentration residue is dissolved in isopropyl alcohol (250 ml). L-(-)-Dibenzoyltartaric acid (20.1 g) is dissolved in water (250 ml). The both mixtures are mixed under heating. The mixture is stood at room temperature for one day and the resulting crystals are collected by filtration. The crystals are recrystallized from a mixed solvent of isopropyl alcohol (370 ml) and water (370 ml) to give 3-amino-1-methyl-5-phenyl-2,3-dihydro-1H-1-benzazepin-2-one D-(+)-dibenzoyltartrate (12.0 g), melting point 170-171°C (dec.).

Example 19

3-Amino-1-methyl-5-phenyl-2,3-dihydro-1H-1-benzazepin-2-one D-(+)-dibenzoyltartrate instead of 3-amino-1-methyl-5-phenyl-2,3-dihydro-1H-1-benzazepin-2-one of Example 7 is made alkaline with 5% aqueous potassium carbonate solution. The mixture is extracted with ethyl acetate and concentrated to give 3-amino-1-methyl-5-phenyl-2,3-dihydro-1H-1-benzazepin-2-one, from which (-)-1-(1-methyl-2-oxo-5-phenyl-2,3-dihydro-1H-1-benzazepin-3-yl)-3-(3-methylphenyl)urea is obtained.
$^1$H-NMR($CDCl_3$); 2.30(3H,s), 3.49(3H,s), 4.72(1H,d), 6.00(1H,d), 6.7-7.6(15H,m), $[\alpha]_D$=-80.0($CHCl_3$,c=0.5)

Example 20

3-Amino-1-methyl-5-phenyl-2,3-dihydro-1H-1-benzazepin-2-one L-(-)-dibenzoyltartrate instead of 3-amino-1-methyl-5-phenyl-2,3-dihydro-1H-1-benzazepin-2-one of Example 7 is made alkaline with 5% aqueous potassium carbonate solution. The mixture is extracted with ethyl acetate and concentrated to give 3-amino-1-methyl-5-phenyl-2,3-dihydro-1H-1-benzazepin-2-one, from which (+)-1-(1-methyl-2-oxo-5-phenyl-2,3-dihydro-1H-1-benzazepin-3-yl)-3-(3-methylphenyl)urea is obtained.
$^1$H-NMR($CDCl_3$); 2.30(3H,s), 3.49(3H,s), 4.72(1H,d), 6.00(1H,d), 6.7-7.6(15H,m), $[\alpha]_D$=+90.2($CHCl_3$,c=0.5)

Example 21

In the same manner as in Example 19, (-)-1-(1-methyl-2-oxo-5-phenyl-2,3-dihydro-1H-1-benzazepin-3-yl)-3-(4-methylphenyl)urea is obtained.
$^1$H-NMR($CDCl_3$); 2.29(3H,s), 3.50(3H,s), 4.70(1H,d), 6.00(1H,d), 6.7-7.5(15H,m), $[\alpha]_D$=-78.9($CHCl_3$,c=0.5)

Example 22

In the same manner as in Example 20, (+)-1-(1-methyl-2-oxo-5-phenyl-2,3-dihydro-1H-1-benzazepin-3-yl)-3-(4-methylphenyl)urea is obtained.
$^1$H-NMR($CDCl_3$); 2.29(3H,s), 3.50(3H,s), 4.70(1H,d), 6.00(1H,d), 6.7-7.5(15H,m), $[\alpha]_D$=+94.9($CHCl_3$,c=0.5)

Example 23

In the same manner as in Example 19, (-)-1-(1-methyl-2-oxo-5-phenyl-2,3-dihydro-1H-1-benzazepin-3-yl)-3-(2-methylphenyl)urea is obtained.
$^{1}$H-NMR($CDCl_3$); 2.30(3H,s), 3.45(3H,s), 4.65(1H,d), 5.97(1H,d), 6.6-7.5(15H,m), $[\alpha]_D$=-58.1($CHCl_3$,c=1.0)

Example 24

In the same manner as in Example 20, (+)-1-(1-methyl-2-oxo-5-phenyl-2,3-dihydro-1H-1-benzazepin-3-yl)-3-(2-methylphenyl)urea is obtained.
$^{1}$H-NMR($CDCl_3$); 2.30(3H,s), 3.45(3H,s), 4.65(1H,d), 5.97(1H,d), 6.6-7.5(15H,m), $[\alpha]_D$=+66.9($CHCl_3$,c=1.0)

Example 25

In the same manner as in Example 19, (-)-1-(4-methoxyphenyl)-3-(1-methyl-2-oxo-5-phenyl-2,3-dihydro-1H-1-benzazepin-3-yl)urea is obtained.
$^{1}$H-NMR($CDCl_3$); 3.46(3H,s), 3.78(3H,s), 4.68(1H,d), 6.00(1H,d), 6.7-7.5(15H,m), $[\alpha]_D$=-67.2($CHCl_3$,c=0.5)

Example 26

In the same manner as in Example 20, (+)-1-(4-methoxyphenyl)-3-(1-methyl-2-oxo-5-phenyl-2,3-dihydro-1H-1-benzazepin-3-yl)urea is obtained.
$^{1}$H-NMR($CDCl_3$); 3.46(3H,s), 3.78(3H,s), 4.68(1H,d), 6.00(1H,d), 6.7-7.5(15H,m), $[\alpha]_D$=+75.4($CHCl_3$,c=0.5)

Example 27

In the same manner as in Example 19, (-)-1-(3-ethoxyphenyl)-3-(1-methyl-2-oxo-5-phenyl-2,3-dihydro-1H-1-benzazepin-3-yl)urea is obtained.
$^{1}$H-NMR($CDCl_3$); 3.49(3H,s), 3.77(3H,s), 4.71(1H,d), 6.01(1H,d), 6.5-7.5(15H,m), $[\alpha]_D$=-83.1($CHCl_3$,c=0.5)

Example 28

In the same manner as in Example 20, (+)-1-(3-methoxyphenyl)-3-(1-methyl-2-oxo-5-phenyl-2,3-dihydro-1H-1-benzazepin-3-yl)urea is obtained.
$^{1}$H-NMR($CDCl_3$); 3.49(3H,s), 3.77(3H,s), 4.71(1H,d), 6.01(1H,d), 6.5-7.5(15H,m), $[\alpha]_D$=+77.5($CHCl_3$,c=0.5)

Example 29

In the same manner as in Example 19, (-)-1-(3-chlorophenyl)-3-(1-methyl-2-oxo-5-phenyl-2,3-dihydro-1H-1-benzazepin-3-yl)urea is obtained.
$^{1}$H-NMR($CDCl_3$); 3.51(3H,s), 4.73(1H,d), 5.99(1H,d), 6.8-7.5(15H,m), $[\alpha]_D$=-80.3($CHCl_3$,c=0.5)

Example 30

In the same manner as in Example 20, (+)-1-(3-chlorophenyl)-3-(1-methyl-2-oxo-5-phenyl-2,3-dihydro-1H-1-benzazepin-3-yl)urea is obtained.
$^{1}$H-NMR($CDCl_3$); 3.51(3H,s), 4.73(1H,d), 5.99(1H,d), 6.8-7.5(15H,m), $[\alpha]_D$=+76.6($CHCl_3$,c=0.5)

Example 31

3-Amino-1-methyl-5-phenyl-2,3-dihydro-1H-1-benzazepin-2-one (0.27 g) prepared in the same manner as in Example 19 is dissolved in tetrahydrofuran (10 ml). Carbodiimidazole (0.23 g) is added and the mixture is stirred at room temperature for one hour. Methyl 3-aminophenylacetate (0.19 g) dissolved in toluene (20 ml) is added and the reaction mixture is heated. After distilling away about 20 ml of the solvent, the mixture is refluxed for 3 hours. The reaction mixture is concentrated under reduced pressure. Water (5 ml) is added to the residue and extracted with ethyl acetate (50 ml). After washing with 0.5N hydrochloric acid and water, the residue is dried over anhydrous magnesium sulfate. The residue is concentrated under reduced pressure and the residue is purified by silica gel column chromatography (developing solvent: chloroform) to give (-)-1-(3-methoxycarbonylmethylphenyl)-3-(1-methyl-2-oxo-5-phenyl-2,3-dihydro-1H-1-benzazepin-3-yl)urea (0.15 g).
$^1$H-NMR($CDCl_3$); 3.50(3H,s), 3.58(2H,s), 3.68(3H,s), 4.71(1H,d), 6.00(1H,d), 6.6-7.5(15H,m), $[\alpha]_D$=-80.4($CHCl_3$,c=0.2)

Example 32

In the same manner as in Example 31 and using 3-amino-1-methyl-5-phenyl-2,3-dihydro-1H-1-benzazepin-2-one prepared in the same manner as in Example 20, (+)-1-(3-methoxycarbonylmethylphenyl)-3-(1-methyl-2-oxo-5-phenyl-2,3-dihydro-1H-1-benzazepin-3-yl)urea is obtained.
$^1$H-NMR($CDCl_3$); 3.50(3H,s), 3.58(2H,s), 3.68(3H,s), 4.71(1H,d), 6.00(1H,d), 6.6-7.5(15H,m), $[\alpha]_D$=+77.6($CHCl_3$,c=0.2)

Example 33

(-)-1-(3-Methoxycarbonylmethylphenyl)-3-(1-methyl-2-oxo-5-phenyl-2,3-dihydro-1H-1-benzazepin-3-yl)urea (0.39 g) is dissolved in water, tetrahydrofuran and dioxane (3 : 4 : 3). 1N Aqueous sodium hydroxide solution (1 ml) is added and the mixture is stirred at room temperature for 1.5 hours. 1N Hydrochloric acid (1.5 ml) is added and the organic solvent is distilled away under reduced pressure. The residue is extracted with ethyl acetate (50 ml). After washing with water, the residue is dried over anhydrous magnesium sulfate and concentrated under reduced pressure to give (-)-1-(3-carboxymethylphenyl)-3-(1-methyl-2-oxo-5-phenyl-2,3-dihydro-1H-1-benzazepin-3-yl)urea (0.25 g).
$^1$H-NMR($CDCl_3$); 3.42(3H,s), 3.58(2H,s), 4.65(1H,d), 5.98(1H,d), 6.7-7.7(16H,m), $[\alpha]_D$=-80.0($CHCl_3$,c=1.0)

Example 34

In the same manner as in Example 33 and using (+)-1-(3-methoxycarbonylmethylphenyl)-3-(1-methyl-2-oxo-2,3-dihydro-1H-1-benzazepin-3-yl)urea instead of (-)-1-(3-methoxycarbonylmethylphenyl)-3-(1-methyl-2-oxo-5-phenyl-2,3-dihydro-1H-1-benzazepin-3-yl)urea, (+)-1-(3-carboxymethylphenyl)-3-(1-methyl-2-oxo-5-phenyl-2,3-dihydro-1H-1-benzazepin-3-yl)urea is obtained.
$^1$H-NMR($CDCl_3$); 3.42(3H,s), 3.58(2H,s), 4.65(1H,d), 5.98(1H,d), 6.7-7.7(16H,m), $[\alpha]_D$=+95.1($CHCl_3$,c=1.0)

Example 35

3-Amino-1-methyl-5-phenyl-2,3-dihydro-1H-1-benzazepin-2-one (0.15 g) prepared in the same manner as in Example 19 and indole-2-carboxylic acid (92 mg) are dissolved in dimethylformamide (5 ml). 1-Hydroxybenzothiazole (92 mg) and 1-(3-dimethylaminopropyl)-3-ethylcarbodiimide hydrochloride (120 mg) are added, and the mixture is stirred at room temperature for one hour. Water (5 ml) is added to the reaction mixture and the mixture is extracted with ethyl acetate (50 ml). After washing with water, the residue is dried over anhydrous magnesium sulfate, concentrated under reduced pressure and purified by silica gel column chromatography (developing solvent: ethyl acetate : hexane = 1 : 2) to give (-)-N-(1-methyl-2-oxo-5-phenyl-2,3-dihydro-1H-1-benzazepin-3-yl)-2-indolecarboxamide (0.14 g).
$^1$H-NMR($CDCl_3$); 3.53(3H,s), 4.84(1H,dd), 6.07(1H,d), 7.1-7.5(14H,m), 7.69(1H,d), 7.88(1H,d), $[\alpha]_D$=-117.1($CHCl_3$,c=0.5)

Example 36

In the same manner as in Example 35 and using 3-amino-1-methyl-5-phenyl-2,3-dihydro-1H-1-benzazepin-2-one prepared in the same manner as in Example 20, (+)-N-(1-methyl-2-oxo-5-phenyl-2,3-dihydro-1H-1-benzazepin-3-yl)-2-indolecarboxamide is obtained.
$^1$H-NMR($CDCl_3$); 3.53(3H,s), 4.84(1H,dd), 6.07(1H,d), 7.1-7.5(14H,m), 7.69(1H,d), 7.88(1H,d), $[\alpha]_D$=+126.0($CHCl_3$,c=0.5)

Example 37

In the same manner as in Example 4 and using ethyl bromoacetate instead of methyl iodide, 1-ethoxycarbonylmethyl-3-phthaloylamino-5-phenyl-2,3,4,5-tetrahydro-1H-1-benzazepin-2-one is obtained.
$^1$H-NMR($CDCl_3$); 1.23(3H,t), 2.04(2H,brs), 3.7-3.8(1H,m), 4.1-4.3(2H,m), 4.52(1H,d), 4.63(1H,d), 5.94(1H,d), 7.1-7.5(9H,m)

Example 38

In the same manner as in Example 5 and using 1-ethoxycarbonylmethyl-3-phthaloylamino-5-phenyl-2,3,4,5-tetrahydro-1H-1-benzazepin-2-one instead of 1-methyl-3-phthaloylamino-5-phenyl-2,3,4,5-tetrahydro-1H-1-benzazepin-2-one, 1-ethoxycarbonylmethyl-3-phthaloylamino-5-phenyl-2,3-dihydro-1H-1-benzazepin-2-one is obtained.
$^1$H-NMR($CDCl_3$); 1.20(3H,t), 4.16(2H,q), 4.56(2H,s), 5.24(1H,d), 6.77(1H,d), 7.0-8.0(13H,m)

Example 39

In the same manner as in Example 6 and using 1-ethoxycarbonylmethyl-3-phthaloylamino-5-phenyl-2,3-dihydro-1H-1-benzazepin-2-one instead of 1-methyl-3-phthaloylamino-5-phenyl-2,3-dihydro-1H-1-benzazepin-2-one, 3-amino-1-ethoxycarbonylmethyl-5-phenyl-2,3-dihydro-1H-1-benzazepin-2-one is obtained.
$^1$H-NMR($CDCl_3$); 1.23(3H,t), 2.04(2H,brs), 3.7-3.8(1H,m), 4.1-4.3(2H,m), 4.52(1H,d), 4.62(1H,d), 5.94(1H,d), 7.1-7.5(9H,m)

Example 40

3-Amino-1-ethoxycarbonylmethyl-5-phenyl-2,3-dihydro-1H-1-benzazepin-2-one (5.43 g) and N-tert-butoxycarbonyl-L-phenylalanine (4.5 g) are dissolved in dimethylformamide (50 ml). 1-Hydroxybenzothiazole (2.3 g) and 1-(3-dimethylaminopropyl)-3-ethylcarbodiimide hydrochloride (3.3 g) are added, and the mixture is stirred at room temperature for 2 hours. 5% Aqueous citric acid solution (25 ml) is added to the reaction mixture and the mixture is extracted with ethyl acetate. After washing with water, the residue is dried over anhydrous magnesium sulfate, concentrated under reduced pressure and purified by silica gel column chromatography (developing solvent: chloroform) to give 1-ethoxycarbonylmethyl-3-(N-tert-butoxycarbonyl-L-phenylalanyl)amino-2-oxo-5-phenyl-2,3-dihydro-1H-1-benzazepine (8.8 g).
$^1$H-NMR($CDCl_3$); 1.1-1.3(3H,m), 1.41(9/2H,s), 1.42(9/2H,s), 3.0-3.2(2H,m), 4.1-4.3(2H,m), 4.4-4.7(4H,m), 4.98(1H,br), 5.61(1/2H,d), 5.80(1/2H,d), 7.1-7.5(15H,m)

Example 41

1-Ethoxycarbonylmethyl-3-(N-tert-butoxycarbonyl-L-phenylalanyl)amino-2-oxo-5-phenyl-2,3-dihydro-1H-1-benzazepine (8.8 g) is dissolved in ethyl acetate, and hydrogen chloride gas is blown in for 2 hours while stirring the mixture at room temperature. The reaction mixture is concentrated under reduced pressure and 5% aqueous potassium carbonate solution is added to make the mixture alkaline. The mixture is extracted with ethyl acetate. After washing with water, the residue is dried over anhydrous magnesium sulfate, concentrated under reduced pressure and purified by silica gel column chromatography (developing solvent: chloroform) to give 1-ethoxycarbonylmethyl-2-oxo-5-phenyl-3-(L-phenylalanyl)-2,3-dihydro-1H-1-benzazepine (3.0 g) eluted first.
$^1$H-NMR($CDCl_3$); 1.20(3H,t), 1.59(2H,br), 2.72(1H,dd), 3.29(1H,dd), 3.7-3.8(1H,m), 4.1-4.3(2H,m), 4.54(1H,d), 4.62(1H,d), 4.74(1H,dd), 5.85(1H,d), 7.2-7.5(14H,m), 8.47(1H,d)

Then, 1-ethoxycarbonylmethyl-2-oxo-5-phenyl-3-(L-phenylalanyl)amino-2,3-dihydro-1H-1-benzazepine (3.2 g) eluted later is obtained.
$^1$H-NMR($CDCl_3$); 1.20(3H,t), 1.57(2H,br), 2.86(1H,dd), 3.26(1H,dd), 3.7-3.8(1H,m), 4.1-4.3(2H,m), 4.54(1H,d), 4.61(1H,d), 4.77(1H,dd), 5.82(1H,d), 7.2-7.5(14H,m), 8.59(1H,d)

Example 42

1-Ethoxycarbonylmethyl-2-oxo-5-phenyl-3-(L-phenylalanyl)amino-2,3-dihydro-1H-1-benzazepine (3.0 g) eluted first in Example 41 is dissolved in dichloromethane (30 ml). Phenylthioisocyanate (0.8 ml) is added and the mixture is stirred at room temperature for 2 hours. The reaction mixture is concentrated under reduced pressure and purified by silica gel column chromatography (developing solvent: chloroform) to give 1-ethoxycarbonylmethyl-2-oxo-5-phenyl-3-(N-phenylaminothiocarbonyl-L-phenylalanyl)amino-2,3-dihydro-1H-1-benzazepine (3.8 g).
$^1$H-NMR($CDCl_3$); 1.19(3H,t), 3.11(1H,dd), 3.39(1H,dd), 4.1-4.3(2H,m), 4.5-4.7(3H,m), 5.33(1H,dd), 5.58(1H,d), 6.79(1H,d), 7.0-7.5(20H,m), 7.88(1H,s)

Example 43

In the same manner as in Example 41 and using 1-ethoxycarbonylmethyl-2-oxo-5-phenyl-3-(L-phenylalanyl)amino-2,3-dihydro-1H-1-benzazepine eluted later in Example 41, instead of 1-ethoxycarbonylmethyl-2-oxo-5-phenyl-3-(L-phenylalanyl)amino-2,3-dihydro-1H-1-benzazepine eluted first in Example 41, 1-ethoxycarbonylmethyl-2-oxo-5-phenyl-3-(N-phenylaminothiocarbonyl-L-phenylalanyl)amino-2,3-dihydro-1H-1-benzazepine, which is an optical isomer of the compound obtained in Example 42, is obtained.
$^1$H-NMR($CDCl_3$); 1.21(3H,t), 3.23(2H,d), 4.1-4.3(2H,m), 4.5-4.7(3H,m), 5.35(1H,dd), 5.84(1H,d), 6.68(1H,d), 7.0-7.5(20H,m), 7.84(1H,s)

Example 44

1-Ethoxycarbonylmethyl-2-oxo-5-phenyl-3-(N-phenylaminothiocarbonyl-L-phenylalanyl)amino-2,3-dihydro-1H-1-benzazepine (3.8 g) obtained in Example 42 is dissolved in trifluoroacetic acid (40 ml), and the mixture is refluxed under heating for 8 hours. The reaction mixture is concentrated under reduced pressure and purified by silica gel column chromatography (developing solvent: chloroform : methanol = 20 : 1) to give 3-amino-1-ethoxycarbonylmethyl-5-phenyl-2,3-dihydro-1H-1-benzazepin-2-one (1.6 g).
$^1$H-NMR($CDCl_3$); 1.23(3H,t), 2.04(2H,brs), 3.7-3.8(1H,m), 4.1-4.3(2H,m), 4.52(1H,d), 4.62(1H,d), 5.94(1H,d), 7.1-7.5(9H,m)

Example 45

In the same manner as in Example 44 and using 1-ethoxycarbonylmethyl-2-oxo-5-phenyl-3-(N-phenylaminothiocarbonyl-L-phenylalanyl)amino-2,3-dihydro-1H-1-benzazepine obtained in Example 43, instead of 1-ethoxycarbonylmethyl-2-oxo-5-phenyl-3-(N-phenylaminothiocarbonyl-L-phenylalanyl)amino-2,3-dihydro-1H-1-benzazepine obtained in Example 42, 3-amino-1-ethoxycarbonylmethyl-5-phenyl-2,3-dihydro-1H-1-benzazepin-2-one is obtained.
$^1$H-NMR($CDCl_3$); 1.23(3H,t), 2.04(2H,brs), 3.7-3.8(1H,m), 4.1-4.3(2H,m), 4.52(1H,d), 4.62(1H,d), 5.94(1H,d), 7.1-7.5(9H,m)

Example 46

Using 3-amino-1-ethoxycarbonylmethyl-5-phenyl-2,3-dihydro-1H-1-benzazepin-2-one obtained in Example 45, instead of 3-amino-1-methyl-5-phenyl-2,3-dihydro-1H-1-benzazepin-2-one of Example 7, (-)-1-(1-ethoxycarbonylmethyl-2-oxo-5-phenyl-2,3-dihydro-1H-1-benzazepin-3-yl)-3-(3-methylphenyl)urea is obtained.
$^1$H-NMR($CDCl_3$); 1.14(3H,t), 2.29(3H,s), 4.1-4.3(2H,m), 4.53(1H,d), 4.64(1H,d), 4.79(1H,dd), 6.00(1H,d), 6.6-7.5(15H,m), $[\alpha]_D$=-74.3($CHCl_3$,c=0.5)

Example 47

Using 3-amino-1-ethoxycarbonylmethyl-5-phenyl-2,3-dihydro-1H-1-benzazepin-2-one obtained in Example 44, instead of 3-amino-1-methyl-5-phenyl-2,3-dihydro-1H-1-benzazepin-2-one of Example 7, (+)-1-(1-ethoxycarbonylmethyl-2-oxo-5-phenyl-2,3-dihydro-1H-1-benzazepin-3-yl)-3-(3-methylphenyl)urea is obtained.
$^1$H-NMR($CDCl_3$); 1.14(3H,t), 2.29(3H,s), 4.1-4.3(2H,m), 4.53(1H,d), 4.64(1H,d), 4.79(1H,dd), 6.00(1H,d), 6.6-7.5(15H,m), $[\alpha]_D$=+75.0($CHCl_3$,c=0.5)

Example 48

In the same manner as in Example 46, (-)-1-(1-ethoxycarbonylmethyl-2-oxo-5-phenyl-2,3-dihydro-1H-1-benzazepin-3-yl)-3-(4- methylphenyl)urea is obtained.
$^1$H-NMR($CDCl_3$); 1.17(3H,t), 2.29(3H,s), 4.1-4.3(2H,m), 4.54(1H,d), 4.63(1H,d), 4.78(1H,d), 5.99(1H,d), 6.7-7.4(15H,m), $[\alpha]_D$=-68.5($CHCl_3$,c=0.5)

Example 49

In the same manner as in Example 47, (+)-1-(1-ethoxycarbonylmethyl-2-oxo-5-phenyl-2,3-dihydro-1H-1-benzazepin-3-yl)-3-(4-methylphenyl)urea is obtained.
$^1$H-NMR($CDCl_3$); 1.17(3H,t), 2.29(3H,s), 4.1-4.3(2H,m), 4.54(1H,d), 4.63(1H,d), 4.78(1H,d), 5.99(1H,d), 6.7-7.4(15H,m), $[\alpha]_D$=+71.0($CHCl_3$,c=0.5)

Example 50

In the same manner as in Example 46, (-)-1-(1-ethoxycarbonylmethyl-2-oxo-5-phenyl-2,3-dihydro-1H-1-benzazepin-3-yl)-3-(2-methylphenyl)urea is obtained.
$^1$H-NMR($CDCl_3$); 1.14(3H,t), 2.28(3H,s), 4.1-4.3(2H,m), 4.50(1H,d), 4.61(1H,d), 4.77(1H,d), 5.96(1H,d), 7.0-7.6(14H,m), $[\alpha]_D$=-52.0($CHCl_3$,c=0.5)

Example 51

In the same manner as in Example 47, (+)-1-(1-ethoxycarbonylmethyl-2-oxo-5-phenyl-2,3-dihydro-1H-1-benzazepin-3-yl)-3-(2-methylphenyl)urea is obtained.
$^1$H-NMR($CDCl_3$); 1.14(3H,t), 2.28(3H,s), 4.1-4.3(2H,m), 4.50(1H,d), 4.61(1H,d), 4.77(1H,d), 5.96(1H,d), 7.0-7.6(14H,m), $[\alpha]_D$=+54.9($CHCl_3$,c=0.5)

Example 52

In the same manner as in Example 46, (-)-1-(1-ethoxycarbonylmethyl-2-oxo-5-phenyl-2,3-dihydro-1H-1-benzazepin-3-yl)-3-(3-methoxyphenyl)urea is obtained.
$^1$H-NMR($CDCl_3$); 1.18(3H,t), 3.76(3H,s), 4.1-4.3(2H,m), 4.54(1H,d), 4.64(1H,d), 4.7-4.9(1H,m), 5.99(1H,d), 6.6-7.4(15H,m), $[\alpha]_D$=68.1($CHCl_3$,c=0.5)

Example 53

In the same manner as in Example 47, (+)-1-(1-ethoxycarbonylmethyl-2-oxo-5-phenyl-2,3-dihydro-1H-1-benzazepin-3-yl)-3-(3-methoxyphenyl)urea is obtained.
$^1$H-NMR($CDCl_3$); 1.18(3H,t), 3.76(3H,s), 4.1-4.3(2H,m), 4.54(1H,d), 4.64(1H,d), 4.7-4.9(1H,m), 5.99(1H,d), 6.6-7.4(15H,m), $[\alpha]_D$=+80.5($CHCl_3$,c=0.5)

Example 54

In the same manner as in Example 46, (-)-1-(3-chlorophenyl)-3-(1-ethoxycarbonylmethyl-2-oxo-5-phenyl-2,3-dihydro-1H-1-benzazepin-3-yl)urea is obtained.
$^1$H-NMR($CDCl_3$); 1.17(3H,t), 4.1-4.3(2H,m), 4.62(2H,d), 4.7-4.9(1H,m), 5.97(1H,d), 6.7-7.5(15H,m), $[\alpha]_D$=-68.3($CHCl_3$,c=0.5)

Example 55

In the same manner as in Example 47, (+)-1-(3-chlorophenyl)-3-(1-ethoxycarbonylmethyl-2-oxo-5-phenyl-2,3-dihydro-1H-1-benzazepin-3-yl)urea is obtained.
$^1$H-NMR($CDCl_3$); 1.17(3H,t), 4.1-4.3(2H,m), 4.62(2H,d), 4.7-4.9(1H,m), 5.97(1H,d), 6.7-7.5(15H,m), $[\alpha]_D$=+70.8($CHCl_3$,c=0.5)

Example 56

In the same manner as in Example 35 and using 3-amino-1-ethoxycarbonylmethyl-5-phenyl-2,3-dihydro-1H-1-benzazepin-2-one obtained in Example 45, instead of 3-amino-1-methyl-5-phenyl-2,3-dihydro-1H-1-benzazepin-2-one, (-)-N-(1-ethoxycarbonylmethyl-2-oxo-5-phenyl-2,3-dihydro-1H-1-benzazepin-3-yl)-2-indolecarboxamide is obtained.
$^1$H-NMR($CDCl_3$); 1.23(3H,t), 4.1-4.3(2H,m), 4.64(2H,s), 4.96(1H,dd), 6.06(1H,d), 7.1-7.9(15H,m), 9.37(1H,s), $[\alpha]_D$=-40.8($CHCl_3$,c=0.5)

Example 57

In the same manner as in Example 56 and using 3-amino-1-ethoxycarbonylmethyl-5-phenyl-2,3-dihydro-1H-1-benzazepin-2-one obtained in Example 44, (+)-N-(1-ethoxycarbonylmethyl-2-oxo-5-phenyl-2,3-dihydro-1H-1-benzazepin-3-yl)-2-indolecarboxamide is obtained.
$^1$H-NMR($CDCl_3$); 1.23(3H,t), 4.1-4.3(2H,m), 4.64(2H,s), 4.96(1H,dd), 6.06(1H,d), 7.1-7.9(15H,m), 9.37(1H,s), $[\alpha]_D$=+64.3($CHCl_3$,c=0.5)

Example 58

In the same manner as in Example 4 and using 2'-methylphenacyl bromide instead of methyl iodide, 1-(2'-methylphenacyl)-3-phthaloylamino-5-phenyl-2,3,4,5-tetrahydro-1H-1-benzazepin-2-one is obtained.
$^1$H-NMR($CDCl_3$); 2.4(3H,s), 2.5-2.9(1H,m), 3.6-4.0(1H,m), 4.8-5.6(4H,m), 6.6-7.9(17H,m)

Example 59

In the same manner as in Example 5 and using 1-(2'-methylphenacyl)-3-phthaloylamino-5-phenyl-2,3,4,5-tetrahydro-1H-1-benzazepin-2-one instead of 1-methyl-3-phthaloylamino-5-phenyl-2,3,4,5-tetrahydro-1H-1-benzazepin-2-one, 1-(2'-methylphenacyl)-3-phthaloylamino-5-phenyl-2,3-dihydro-1H-1-benzazepin-2-one is obtained.
$^1$H-NMR($CDCl_3$); 2.4(3H,s), 4.8-5.6(3H,m), 6.6-8.0(18H,m)

Example 60

In the same manner as in Example 6 and using 1-(2'-methylphenacyl)-3-phthaloylamino-5-phenyl-2,3-dihydro-1H-1-benzazepin-2-one instead of 1-methyl-3-phthaloylamino-5-phenyl-2,3-dihydro-1H-1-benzazepin-2-one, 3-amino-1-(2'-methylphenacyl)-5-phenyl-2,3-dihydro-1H-1-benzazepin-2-one is obtained.
$^1$H-NMR($CDCl_3$); 2.26(2H,br), 2.49(3H,s), 3.79(1H,d), 5.06(1H,d), 5.26(1H,d), 5.93(1H,d), 7.0-7.7(13H,m)

Example 61

3-Amino-1-(2'-methylphenacyl)-5-phenyl-2,3-dihydro-1H-1-benzazepin-2-one (19.4 g) and L-(+)-mandelic acid (6.9 g) are dissolved in toluene (245 ml) under heating. The mixture is left standing at room temperature for one day. The resulting crystals are collected by filtration to give 3-amino-1-(2'-methylphenacyl)-5-phenyl-2,3-dihydro-1H-1-benzazepin-2-one L-(+)-mandelate (9.0 g), melting point 135-136°C (dec.). The filtrate is concentrated under reduced pressure. 5% Aqueous potassium carbonate solution is added to make the mixture alkaline, and the mixture is extracted with chloroform (500 ml). After washing with water, the residue is dried over anhydrous magnesium sulfate, and concentrated under reduced pressure. The concentration residue and D-(-)-mandelic acid (5.2 g) are dissolved in toluene (186 ml). The mixture is left standing at room temperature for one day. The resulting crystals are collected by filtration to give 3-amino-1-(2'-methylphenacyl)-5-phenyl-2,3-dihydro-1H-1-benzazepin-2-one D-(-)-mandelate (2.0 g), melting point 134-135°C (dec.).

Example 62

3-Amino-1-(2'-methylphenacyl)-5-phenyl-2,3-dihydro-1H-1-benzazepin-2-one D-(-)-mandelate is made alkaline with 5% aqueous potassium carbonate solution. The mixture is extracted with ethyl acetate and concentrated to give 3-amino-1-(2'-methylphenacyl)-5-phenyl-2,3-dihydro-1H-1-benzazepin-2-one. The obtained compound is used instead of 3-amino-1-methyl-5-phenyl-2,3-dihydro-1H-1-benzazepin-2-one of Example 7 to give (-)-1-(1-(2'-methylphenacyl)-2-oxo-5-phenyl-2,3-dihydro-1H-1-benzazepin-3-yl)-3-(3-methylphenyl)urea.
$^1$H-NMR($CDCl_3$); 2.28(3H,s), 2.45(3H,s), 4.81(1H,d), 5.16(1H,d), 5.21(1H,d), 6.0(1H,d), 6.56(1H,br), 6.85(1H,d), 7.0-7.4(15H,m), 7.63(1H,d), $[\alpha]_D$=35.2($CHCl_3$,c=0.5)

Example 63

3-Amino-1-(2'-methylphenacyl)-5-phenyl-2,3-dihydro-1H-1-benzazepin-2-one L-(+)-mandelate is made alkaline with 5% aqueous potassium carbonate solution. The mixture is extracted with ethyl acetate and concentrated to give 3-amino-1-(2'-methylphenacyl)-5-phenyl-2,3-dihydro-1H-1-benzazepin-2-one. The obtained compound is used instead of 3-amino-1-methyl-5-phenyl-2,3-dihydro-1H-1-benzazepin-2-one of Example 7 to give (+)-1-(1-(2'-methylphenacyl)-2-oxo-5-phenyl-2,3-dihydro-1H-1-benzazepin-3-yl)-3-(3-methylphenyl)urea.
$^1$H-NMR($CDCl_3$); 2.28(3H,s), 2.45(3H,s), 4.81(1H,d), 5.16(1H,d), 5.21(1H,d), 6.0(1H,d), 6.56(1H,br), 6.85(1H,d), 7.0-7.4(15H,m), 7.63(1H,d), $[\alpha]_D$=+30.7($CHCl_3$,c=0.5)

Example 64

In the same manner as in Example 62, (-)-1-(1-(2'-methylphenacyl)-2-oxo-5-phenyl-2,3-dihydro-1H-1-benzazepin-3-yl)-3-(4-methylphenyl)urea is obtained.
$^1$H-NMR($CDCl_3$); 2.28(3H,s), 2.44(3H,s), 4.81(1H,d), 5.14(1H,d), 5.21(1H,d), 5.99(1H,d), 6.74(1H,brs), 7.0-7.7(18H,m), $[\alpha]_D$=-27.2($CHCl_3$,c=0.5)

Example 65

In the same manner as in Example 63, (+)-1-(1-(2'-methylphenacyl)-2-oxo-5-phenyl-2,3-dihydro-1H-1-benzazepin-3-yl)-3-(4-methylphenyl)urea is obtained.

$^1H$-NMR($CDCl_3$); 2.28(3H,s), 2.44(3H,s), 4.81(1H,d), 5.14(1H,d), 5.21(1H,d), 5.99(1H,d), 6.74(1H,brs), 7.0-7.7(18H,m), $[\alpha]_D$=+31.1($CHCl_3$,c=0.5)

Example 66

In the same manner as in Example 62, (-)-1-(1-(2'-methylphenacyl)-2-oxo-5-phenyl-2,3-dihydro-1H-1-benzazepin-3-yl)-3-(2-methylphenyl)urea is obtained.
$^1H$-NMR($CDCl_3$); 2.26(3H,s), 2.41(3H,s), 4.79(1H,d), 5.11(1H,d), 5.20(1H,d), 5.97(1H,d), 6.54(1H,brs), 7.0-7.7(18H,m), $[\alpha]_D$=-19.4($CHCl_3$,c=1.0)

Example 67

In the same manner as in Example 63, (+)-1-(1-(2'-methylphenacyl)-2-oxo-5-phenyl-2,3-dihydro-1H-1-benzazepin-3-yl)-3-(2-methylphenyl)urea is obtained.
$^1H$-NMR($CDCl_3$); 2.26(3H,s), 2.41(3H,s), 4.79(1H,d), 5.11(1H,d), 5.20(1H,d), 5.97(1H,d), 6.54(1H,brs), 7.0-7.7(18H,m), $[\alpha]_D$=+22.3($CHCl_3$,c=1.0)

Example 68

In the same manner as in Example 62, (-)-1-(4-methoxyphenyl)-3-(1-(2'-methylphenacyl)-2-oxo-5-phenyl-2,3-dihydro-1H-1-benzazepin-3- yl)urea is obtained.
$^1H$-NMR($CDCl_3$); 2.24(3H,s), 3.75(3H,s), 4.80(1H,dd), 5.12(1H,d), 5.20(1H,d), 5.98(1H,d), 6.53(1H,d), 6.7-7.4(17H,m), 7.61(1H,d), $[\alpha]_D$=-19.2($CHCl_3$,c=0.5)

Example 69

In the same manner as in Example 63, (+)-1-(4-methoxyphenyl)-3-(1-(2'-methylphenacyl)-2-oxo-5-phenyl-2,3-dihydro-1H-1-benzazepin-3-yl)urea is obtained.
$^1H$-NMR($CDCl_3$); 2.42(3H,s), 3.75(3H,s), 4.80(1H,dd), 5.12(1H,d), 5.20(1H,d), 5.98(1H,d), 6.53(1H,d), 6.7-7.4(17H,m), 7.61(1H,d), $[\alpha]_D$=+21.2($CHCl_3$,c=0.5)

Example 70

In the same manner as in Example 62, (-)-1-(3-methoxyphenyl)-3-(1-(2'-methylphenacyl)-2-oxo-5-phenyl-2,3-dihydro-1H-1-benzazepin-3-yl)urea is obtained.
$^1H$-NMR($CDCl_3$); 2.43(3H,s), 3.73(3H,s), 4.83(1H,dd), 5.18(2H,s), 6.00(1H,d), 6.5-7.7(19H,m), $[\alpha]_D$=-29.8($CHCl_3$,c=0.5)

Example 71

In the same manner as in Example 63, (+)-1-(3-methoxyphenyl)-3-(1-(2'-methylphenacyl)-2-oxo-5-phenyl-2,3-dihydro-1H-1-benzaaepin-3-yl)urea is obtained.
$^1H$-NMR($CDCl_3$); 2.43(3H,s), 3.73(3H,s), 4.83(1H,dd), 5.18(2H,s), 6.00(1H,d), 6.5-7.7(19H,m), $[\alpha]_D$=+27.0($CHCl_3$,c=0.5)

Example 72

In the same manner as in Example 62, (-)-1-(3-chlorophenyl)-3-(1-(2'-methylphenacyl)-2-oxo-5-phenyl-2,3-dihydro-1H-1-benzazepin-3-yl)urea is obtained.
$^1H$-NMR($CDCl_3$); 2.46(3H,s), 4.85(1H,m), 5.97(1H,d), 6.8-7.7(19H,m), $[\alpha]_D$=-18.5($CHCl_3$,c=0.5)

Example 73

In the same manner as in Example 63, (+)-1-(3-chlorophenyl)-3-(1-(2'-methylphenacyl)-2-oxo-5-phenyl-2,3-dihydro-1H-1-benzazepin-3- yl)urea is obtained.
$^1H$-NMR($CDCl_3$); 2.46(3H,s), 4.85(1H,m), 5.97(1H,d), 6.8-7.7(19H,m), $[\alpha]_D$=+19.9($CHCl_3$,c=0.5)

Example 74

In the same manner as in Example 62, (-)-1-(2-chlorophenyl)-3-(1-(2'-methylphenacyl)-2-oxo-5-phenyl-2,3-dihydro-1H-1-benzazepin-3-yl)urea is obtained.
$^1$H-NMR($CDCl_3$); 2.47(3H,s), 4.80(1H,t), 5.20(2H,s), 6.03(1H,d), 6.58(1H,d), 6.8-7.4(16H,m), 7.66(1H,dd), 8.11(1H,dd), $[\alpha]_D$=-40.4($CHCl_3$,c=0.5)

Example 75

In the same manner as in Example 63, (+)-1-(2-chlorophenyl)-3-(1-(2'-methylphenacyl)-2-oxo-5-phenyl-2,3-dihydro-1H-1-benzazepin-3-yl)urea is obtained.
$^1$H-NMR($CDCl_3$); 2.47(3H,s), 4.80(1H,t), 5.20(2H,s), 6.03(1H,d), 6.58(1H,d), 6.8-7.4(16H,m), 7.66(1H,dd), 8.11(1H,dd), $[\alpha]_D$=+35.3($CHCl_3$,c=0.5)

Example 76

In the same manner as in Example 31 and using 3-amino-1-(2'-methylphenacyl)-5-phenyl-2,3-dihydro-1H-1-benzazepin-2-one prepared in the same manner as in Example 62 and methyl 3-aminophenylacetate, (-)-1-(3-methoxycarbonylmethylphenyl)-3-(1-(2'-methylphenacyl)-2-oxo-5-phenyl-2,3-dihydro-1H-1-benzazepin-3-yl)urea is obtained.
$^1$H-NMR($CDCl_3$); 2.45(3H,s), 3.55(2H,s), 3.66(3H,s), 4.80(1H,d), 5.16(1H,d), 5.24(1H,d), 6.70(1H,br), 6.93(1H,d), 6.98(1H,br), 7.1-7.4(15H,m), 7.64(1H,d), $[\alpha]_D$=-25.0($CHCl_3$,c=0.5)

Example 77

In the same manner as in Example 31 and using 3-amino-1-(2'-methylphenacyl)-5-phenyl-2,3-dihydro-1H-1-benzazepin-2-one prepared in the same manner as in Example 63 and methyl 3-aminophenylacetate, (+)-1-(3-methoxycarbonylmethylphenyl)-3-(1-(2'-methylphenacyl)-2-oxo-5-phenyl-2,3-dihydro-1H-1-benzazepin-3-yl)urea is obtained.
$^1$H-NMR($CDCl_3$); 2.45(3H,s), 3.55(2H,s), 3.66(3H,s), 4.80(1H,d), 5.16(1H,d), 5.24(1H,d), 6.70(1H,br), 6.93(1H,d), 6.98(1H,br), 7.1-7.4(15H,m), 7.64(1H,d), $[\alpha]_D$=+22.2($CHCl_3$,c=0.5)

Example 78

In the same manner as in Example 33 and using (-)-1-(3-methoxycarbonylmethylphenyl)-3-(1-(2'-methylphenacyl)-2-oxo-5-phenyl-2,3-dihydro-1H-1-benzazepin-3-yl)urea, (-)-1-(3-carboxymethylphenyl)-3-(1-(2'-methylphenacyl)-2-oxo-5-phenyl-2,3-dihydro-1H-1-benzazepin-3-yl)urea is obtained.
$^1$H-NMR($CDCl_3$); 2.44(3H,s), 3.59(2H,s), 4.81(1H,d), 5.12(1H,d), 5.23(1H,d), 5.99(1H,d), 6.8-7.8(19H,m), $[\alpha]_D$=-38.0($CHCl_3$,c=1.0)

Example 79

In the same manner as in Example 33 and using (+)-1-(3-methoxycarbonylmethylphenyl)-3-(1-(2'-methylphenacyl)-2-oxo-5-phenyl-2,3-dihydro-1H-1-benzazepin-3-yl)urea, (+)-1-(3-carboxymethylphenyl)-3-(1-(2'-methylphenacyl)-2-oxo-5-phenyl-2,3-dihydro-1H-1-benzazepin-3-yl)urea is obtained.
$^1$H-NMR($CDCl_3$); 2.44(3H,s), 3.59(2H,s), 4.81(1H,d), 5.12(1H,d), 5.23(1H,d), 5.99(1H,d), 6.8-7.8(19H,m), $[\alpha]_D$=+31.4($CHCl_3$,c=1.0)

Example 80

In the same manner as in Example 35 and using 3-amino-1-(2'-methylphenacyl)-5-phenyl-2,3-dihydro-1H-1-benzazepin-2-one prepared in the same manner as in Example 62, (-)-N-(1-(2'-methylphenacyl)-2-oxo-5-phenyl-2,3-dihydro-1H-1-benzazepin-3-yl)-2-indolecarboxamide (0.14 g) is obtained.
$^1$H-NMR($CDCl_3$); 2.50(3H,s), 4.98(1H,d), 5.18(1H,d), 5.27(1H,d), 6.06(1H,d), 7.00-7.72(20H,m), $[\alpha]_D$=-36.2($CHCl_3$,c=0.5)

Example 81

In the same manner as in Example 35 and using 3-amino-1-(2'-methylphenacyl)-5-phenyl-2,3-dihydro-1H-1-benzazepin-2-one prepared in the same manner as in Example 63, (+)-N-(1-(2'-methylphenacyl)-2-oxo-5-phenyl-2,3-dihydro-1H-1-benzazepin-3-yl)-2-indole carboxamide is obtained.

$^1H$-NMR($CDCl_3$); 2.50(3H,s), 4.98(1H,d), 5.18(1H,d), 5.27(1H,d), 6.06(1H,d), 7.00-7.72(20H,m), $[\alpha]_D$=+49.4($CHCl_3$,c=0.5)

Example 82

In the same manner as in Example 37, 3-phthaloylamino-5-phenyl-1-isopropoxycarbonylmethyl-2,3,4,5-tetrahydro-1H-1-benzazepin-2-one is obtained.

Example 83

In the same manner as in Example 38, 3-phthaloylamino-5-phenyl-1-isopropoxycarbonylmethyl-2,3-dihydro-1H-1-benzazepin-2-one is obtained.

Example 84

In the same manner as in Example 39, 3-amino-5-phenyl-1-isopropoxycarbonylmethyl-2,3-dihydro-1H-1-benzazepin-2-one is obtained.

Example 85

In the same manner as in Example 46, 1-(3-methylphenyl)-3-(2-oxo-5-phenyl-1-isopropoxycarbonylmethyl-2,3-dihydro-1H-1-benzazepin-3-yl)urea is obtained.

Example 86

In the same manner as in Example 48, 1-(4-methylphenyl)-3-(2-oxo-5-phenyl-1-isopropoxycarbonylmethyl-2,3-dihydro-1H-1-benzazepin-3-yl)urea is obtained.

Example 87

In the same manner as in Example 50, 1-(2-methylphenyl)-3-(2-oxo-5-phenyl-1-isopropoxycarbonylmethyl-2,3-dihydro-1H-1-benzazepin-3-yl)urea is obtained.

Example 88

In the same manner as in Example 52, 1-(2-methoxyphenyl)-3-(2-oxo-5-phenyl-1-isopropoxycarbonylmethyl-2,3-dihydro-1H-1-benzazepln-3-yl)urea is obtained.

Example 89

In the same manner as in Example 88, 1-(3-methoxyphenyl)-3-(2-oxo-5-phenyl-1-isopropoxycarbonylmethyl-2,3-dihydro-1H-1- benzazepin-3-yl)urea is obtained.

Example 90

In the same manner as in Example 88, 1-(4-methoxyphenyl)-3-(2-oxo-5-phenyl-1-isopropoxycarbonylmethyl-2,3-dihydro-1H-1-benzazepin-3-yl)urea is obtained.

Example 91

In the same manner as in Example 54, 1-(3-chlorophenyl)-3-(2-oxo-5-phenyl-1-isopropoxycarbonylmethyl-2,3-dihydro-1H-1-benzazepin-3-yl)urea is obtained.

Example 92

In the same manner as in Example 91, 1-(2-chlorophenyl)-3-(2-oxo-5-phenyl-1-isopropoxycarbonylmethyl-2,3-dihydro-1H-1-benzazepin-3-yl)urea is obtained.

Example 93

In the same manner as in Example 91, 1-(4-chlorophenyl)-3-(2-oxo-5-phenyl-1-isopropoxycarbonylmethyl-2,3-dihydro-1H-1-benzazepin-3-yl)urea is obtained.

Example 94

In the same manner as in Example 37, 1-tert-butoxycarbonylmethyl-3-phthaloylamino-5-phenyl-2,3,4,5-tetrahydro-1H-1-benzazepin-2-one is obtained.
$^1$H-NMR($CDCl_3$); 1.45(9H,s), 2.55-2.75(1H,m), 3.70-3.85(1H,m), 4.35(1H,d), 4.70(1H,d), 4.90-5.15(2H,m), 6.70-7.50(9H,m), 7.64-7.90(4H,m)

Example 95

In the same manner as in Example 38, 1-tert-butoxycarbonylmethyl-3-phthaloylamino-5-phenyl-2,3-dihydro-1H-1-benzazepin-2-one is obtained.
$^1$H-NMR($CDCl_3$); 1.41(9H,s), 4.37(1H,d), 4.54(1H,d), 5.25(1H,d), 6.82(1H,d), 7.10-7.50(9H,m), 7.65-7.95(4H,m)

Example 96

In the same manner as in Example 39, 3-amino-1-tert-butoxycarbonylmethyl-5-phenyl-2,3-dihydro-1H-1-benzazepin-2-one is obtained.
$^1$H-NMR($CDCl_3$); 1.47(9H,s), 2.04(2H,brs), 3.72(1H,d), 4.32(1H,d), 4.57(1H,d), 5.93(1H,d), 7.00-7.60(9H,m)

Example 97

In the same manner as in Example 46, 1-(1-tert-butoxycarbonylmethyl-2-oxo-5-phenyl-2,3-dihydro-1H-1-benzazepin-3-yl)-3-(3-methylphenyl)urea is obtained.
$^1$H-NMR($CDCl_3$); 1.41(9H,s), 2.29(3H,s), 4.48(2H,s), 4.7-4.8(1H,m), 6.02(1H,d), 6.50-6.65(1H,br), 6.8-7.45(14H,m)

Example 98

In the same manner as in Example 48, 1-(1-tert-butoxycarbonylmethyl-2-oxo-5-phenyl-2,3-dihydro-1H-1-benzazepin-3-yl)-3-(4-methylphenyl)urea is obtained.

Example 99

In the same manner as in Example 50, 1-(1-tert-butoxycarbonylmethyl-2-oxo-5-phenyl-2,3-dihydro-1H-1-benzazepin-3-yl)-3-(2-methylphenyl)urea is obtained.

Example 100

In the same manner as in Example 52, 1-(1-tert-butoxycarbonylmethyl-2-oxo-5-phenyl-2,3-dihydro-1H-1-benzazepin-3-yl)-3-(2-methoxyphenyl)urea is obtained.

Example 101

In the same manner as in Example 88, 1-(1-tert-butoxycarbonylmethyl-2-oxo-5-phenyl-2,3-dihydro-1H-1-benzazepin-3-yl)-3-(3-methoxyphenyl)urea is obtained.

Example 102

In the same manner as in Example 88, 1-(1-tert-butoxycarbonylmethyl-2-oxo-5-phenyl-2,3-dihydro-1H-1-benzazepin-3-yl)-3-(4-methoxyphenyl)urea is obtained.

Example 103

In the same manner as in Example 54, 1-(1-tert-butoxycarbonylmethyl-2-oxo-5-phenyl-2,3-dihydro-1H-1-benzazepin-3-yl)-3-(3- chlorophenyl)urea is obtained.

Example 104

In the same manner as in Example 91, 1-(1-tert-butoxycarbonylmethyl-2-oxo-5-phenyl-2,3-dihydro-1H-1-benzazepin-3-yl)-3-(2-chlorophenyl)urea is obtained.

Example 105

In the same manner as in Example 91, 1-(1-tert-butoxycarbonylmethyl-2-oxo-5-phenyl-2,3-dihydro-1H-1-benzazepin-3-yl)-3-(4-chlorophenyl)urea is obtained.

Example 106

In the same manner as in Example 31, 1-(3-methoxycarbonyl-methylphenyl)-3-(2-oxo-5-phenyl-1-isopropoxycarbonylmethyl-2,3-dihydro-1H-1-benzazepin-3-yl)urea is obtained.

Example 107

In the same manner as in Example 31, 1-(1-tert-butoxycarbonylmethyl-2-oxo-5-phenyl-2,3-dihydro-1H-1-benzazepin-3-yl)-3-(3-methoxycarbonylmethylphenyl)urea is obtained.

Example 108

In the same manner as in Example 35, N-(2-oxo-5-phenyl-1-isopropoxycarbonylmethyl-2,3-dihydro-1H-1-benzazepin-3-yl)-2-indolecarboxamide is obtained.

Example 109

In the same manner as in Example 35, N-(1-tert-butoxycarbonylmethyl-2-oxo-5-phenyl-2,3-dihydro-1H-1-benzazepin-3-yl)-2-indolecarboxamide is obtained.

Example 110

In the same manner as in Example 37, 1-tert-butylaminocarbonylmethyl-3-phthaloylamino-5-phenyl-2,3,4,5-tetrahydro-1H-1-benzazepin-2-one is obtained.
$^1$H-NMR($CDCl_3$); 1.34(9H,s), 2.60-2.80(1H,m), 3.75-3.94(1H,m), 4.18(1H,d), 4.65(1H,d), 4.60-4.75(1H,m), 4.90-5.10(1H,m), 6.70-7.50(10H,m), 7.70-7.90(4H,m)

Example 111

In the same manner as in Example 38, 1-tert-butylaminocarbonylmethyl-3-phthaloylamino-5-phenyl-2,3-dihydro-1H-1-benzazepin-2-one is obtained.

Example 112

In the same manner as in Example 39, 3-amino-1-tert-butylaminocarbonylmethyl-5-phenyl-2,3-dihydro-1H-1-benzazepin-2-one is obtained.

Example 113

In the same manner as in Example 46, 1-(1-tert-butylaminocarbonylmethyl-2-oxo-5-phenyl-2,3-dihydro-1H-1-benzazepin-3-yl)-3-(3-methylphenyl)urea is obtained.

Example 114

In the same manner as in Example 48, 1-(1-tert-butylaminocarbonylmethyl-2-oxo-5-phenyl-2,3-dihydro-1H-1-benzazepin-3-yl)-3-(4-methylphenyl)urea is obtained.

Example 115

In the same manner as in Example 50, 1-(1-tert-butylaminocarbonylmethyl-2-oxo-5-phenyl-2,3-dihydro-1H-1-benzazepin-3-yl)-3-(2-methylphenyl)urea is obtained.

Example 116

In the same manner as in Example 52, 1-(1-tert-butylaminocarbonylmethyl-2-oxo-5-phenyl-2,3-dihydro-1H-1-benzazepin-3-yl)-3-(2-methoxyphenyl)urea is obtained.

Example 117

In the same manner as in Example 88, 1-(1-tert-butylaminocarbonylmethyl-2-oxo-5-phenyl-2,3-dihydro-1H-1-benzazepin-3-yl)-3-(3-methoxyphenyl)urea is obtained.

Example 118

In the same manner as in Example 88, 1-(1-tert-butylaminocarbonylmethyl-2-oxo-5-phenyl-2,3-dihydro-1H-1-benzazepin-3-yl)-3-(4-methoxyphenyl)urea is obtained.

Example 119

In the same manner as in Example 54, 1-(1-tert-butylaminocarbonylmethyl-2-oxo-5-phenyl-2,3-dihydro-1H-1-benzazepin-3-yl)-3- (3-chlorophenyl)urea is obtained.

Example 120

In the same manner as in Example 91, 1-(1-tert-butylaminocarbonylmethyl-2-oxo-5-phenyl-2,3-dihydro-1H-1-benzazepin-3-yl)-3-(2-chlorophenyl)urea is obtained.

Example 121

In the same manner as in Example 91, 1-(1-tert-butylaminocarbonylmethyl-2-oxo-5-phenyl-2,3-dihydro-1H-1-benzazepin-3-yl)-3-(4-chlorophenyl)urea is obtained.

Example 122

In the same manner as in Example 31, 1-(1-tert-butylaminocarbonylmethyl-2-oxo-5-phenyl-2,3-dihydro-1H-1-benzazepin-3-yl)-3-(3-methoxycarbonylmethylphenyl)urea is obtained.

Example 123

In the same manner as in Example 35, N-(1-tert-butylaminocarbonylmethyl-2-oxo-5-phenyl-2,3-dihydro-1H-1-benzazepin-3-yl)-2-indolecarboxamide is obtained.

Example 124

In the same manner as in Example 37, 1-diethylaminocarbonylmethyl-3-phthaloylamino-5-phenyl-2,3,4,5-tetrahydro-1H-1-benzazepin2-one is obtained.

Example 125

In the same manner as in Example 38, 1-diethylaminocarbonyl-methyl-3-phthaloylamino-5-phenyl-2,3-dihydro-1H-1-benzazepin-2-one is obtained.

Example 126

In the same manner as in Example 39, 1-diethylaminocarbonylmethyl-3-amino-5-phenyl-2,3-dihydro-1H-1-benzazepin-2-one is obtained.

Example 127

In the same manner as in Example 46, 1-(1-diethylaminocarbonylmethyl-2-oxo-5-phenyl-2,3-dihydro-1H-1-benzazepin-3-yl)-3-(3- methylphenyl)urea is obtained.

Example 128

In the same manner as in Example 48, 1-(1-diethylaminocarbonylmethyl-2-oxo-5-phenyl-2,3-dihydro-1H-1-benzazepin-3-yl)-3-(4-methylphenyl)urea is obtained.

Example 129

In the same manner as in Example 50, 1-(1-diethylaminocarbonylmethyl-2-oxo-5-phenyl-2,3-dihydro-1H-1-benzazepin-3-yl)-3-(2-methylphenyl)urea is obtained.

Example 130

In the same manner as in Example 52, 1-(1-diethylaminocarbonylmethyl-2-oxo-5-phenyl-2,3-dihydro-1H-1-benzazepin-3-yl)-3-(2-methoxyphenyl)urea is obtained.

Example 131

In the same manner as in Example 88, 1-(1-diethylaminocarbonylmethyl-2-oxo-5-phenyl-2,3-dihydro-1H-1-benzazepin-3-yl)-3-(3-methoxyphenyl)urea is obtained.

Example 132

In the same manner as in Example 88, 1-(1-diethylaminocarbonylmethyl-2-oxo-5-phenyl-2,3-dihydro-1H-1-benzazepin-3-yl)-3-(4-methoxyphenyl)urea is obtained.

Example 133

In the same manner as in Example 54, 1-(1-diethylaminocarbonylmethyl-2-oxo-5-phenyl-2,3-dihydro-1H-1-benzazepin-3-yl)-3-(3-chlorophenyl)urea is obtained.

Example 134

In the same manner as in Example 91, 1-(1-diethylaminocarbonylmethyl-2-oxo-5-phenyl-2,3-dihydro-1H-1-benzazepin-3-yl)-3-(2-chlorophenyl)urea is obtained.

Example 135

In the same manner as in Example 91, 1-(1-diethylaminocarbonylmethyl-2-oxo-5-phenyl-2,3-dihydro-1H-1-benzazepin-3-yl)-3-(4-chlorophenyl)urea is obtained.

Example 136

In the same manner as in Example 31, 1-(1-diethylaminocarbonylmethyl-2-oxo-5-phenyl-2,3-dihydro-1H-1-benzazepin-3-yl)-3-(3-methoxycarbonylmethylphenyl)urea is obtained.

Example 137

In the same manner as in Example 35, N-(1-diethylaminocarbonylmethyl-2-oxo-5-phenyl-2,3-dihydro-1H-1-benzazepin-3-yl)-2-indolecarboxamide is obtained.

Example 138

In the same manner as in Example 37, 1-(pyrrolidin-1-ylcarbonylmethyl)-3-phthaloylamino-5-phenyl-2,3,4,5-tetrahydro-1H-1-benzazepin-2-one is obtained.

Example 139

In the same manner as in Example 38, 1-(pyrrolidin-1-ylcarbonylmethyl)-3-phthaloylamino-5-phenyl-2,3-dihydro-1H-1-benzazepin-2-one is obtained.

Example 140

In the same manner as in Example 39, 3-amino-5-phenyl-1-(pyrrolidin-1-ylcarbonylmethyl)-2,3-dihydro-1H-1-benzazepin-2-one is obtained.

Example 141

In the same manner as in Example 46, 1-(3-methylphenyl)-3-(2-oxo-5-phenyl-1-(pyrrolidin-1-ylcarbonylmethyl)-2,3-dihydro-1H-1-benzazepin-3-yl)urea is obtained.

Example 142

In the same manner as in Example 48, 1-(4-methylphenyl)-3-(2-oxo-5-phenyl-1-(pyrrolidin-1-ylcarbonylmethyl)-2,3-dihydro-1H-1-benzazepin-3-yl)urea is obtained.

Example 143

In the same manner as in Example 50, 1-(2-methylphenyl)-3-(2-oxo-5-phenyl-1-(pyrrolidin-1-ylcarbonylmethyl)-2,3-dihydro-1H-1-benzazepin-3-yl)urea is obtained.

Example 144

In the same manner as in Example 52, 1-(2-methoxyphenyl)-3-(2-oxo-5-phenyl-1-(pyrrolidin-1-ylcarbonylmethyl)-2,3-dihydro-1H-1-benzazepin-3-yl)urea is obtained.

Example 145

In the same manner as in Example 88, 1-(3-methoxyphenyl)-3-(2-oxo-5-phenyl-1-(pyrrolidin-1-ylcarbonylmethyl)-2,3-dihydro-1H-1-benzazepin-3-yl)urea is obtained.

Example 146

In the same manner as in Example 88, 1-(4-methoxyphenyl)-3-(2-oxo-5-phenyl-1-(pyrrolidin-1-ylcarbonylmethyl)-2,3-dihydro-1H-1-benzazepin-3-yl)urea is obtained.

Example 147

In the same manner as in Example 54, 1-(3-chlorophenyl)-3-(2-oxo-5-phenyl-1-(pyrrolidin-1-ylcarbonylmethyl)-2,3-dihydro-1H-1-benzazepin-3-yl)urea is obtained.

Example 148

In the same manner as in Example 91, 1-(2-chlorophenyl)-3-(2-oxo-5-phenyl-1-(pyrrolidin-1-ylcarbonylmethyl)-2,3-dihydro-1H-1-benzazepin-3-yl)urea is obtained.

Example 149

In the same manner as in Example 91, 1-(4-chlorophenyl)-3-(2-oxo-5-phenyl-1-(pyrrolidin-1-ylcarbonylmethyl)-2,3-dihydro-1H-1-benzazepin-3-yl)urea is obtained.

Example 150

In the same manner as in Example 31, 1-(3-methoxycarbonyl-methylphenyl)-3-(2-oxo-5-phenyl-1-(pyrrolidin-1-ylcarbonylmethyl)-2,3-dihydro-1H-1-benzazepin-3-yl)urea is obtained.

Example 151

In the same manner as in Example 35, N-(1-(pyrrolidin-1-ylcarbonylmethyl)-2-oxo-5-phenyl-2,3-dihydro-1H-1-benzazepin-3-yl)-2-indolecarboxamide is obtained.

Example 152

From 3-amino-1-tert-butoxycarbonylmethyl-5-phenyl-2,3-dihydro-1H-1-benzazepin-2-one obtained in Example 96, two kinds of optically active compounds are obtained by high performance liquid chromatography (column: ULTRON ES-OVM, solvent: 1/15M potassium dihydrogenphosphate aqueous solution, 1/15M disodium hydrogenphosphate aqueous solution, acetonitrile).

Example 153

In the same manner as in Example 35 and using 3-amino-1-tert-butoxycarbonylmethyl-5-phenyl-2,3-dihydro-1H-1-benzazepin-2-one eluted first in Example 152, (+)-N-(1-tert-butoxycarbonylmethyl-2-oxo-5-phenyl-2,3-dihydro-1H-1-benzazepin-3-yl)-2-indolecarboxamide is obtained.

Example 154

In the same manner as in Example 152, two kinds of optically active compounds of 3-amino-5-phenyl-1-isopropoxycarbonylmethyl-2,3-dihydro-1H-1-benzazepin-2-one are obtained.

Example 155

In the same manner as in Example 35 and using 3-amino-5-phenyl-1-isopropoxycarbonylmethyl-2,3-dihydro-1H-1-benzazepin-2-one eluted first in Example 154, (+)-N-(1-isopropoxycarbonylmethyl-2-oxo-5-phenyl-2,3-dihydro-1H-1-benzazepin-3-yl)-2-indolecarboxamide is obtained.

Example 156

In the same manner as in Example 152, two kinds of optically active compounds of 3-amino-1-tert-butylaminocarbonylmethyl-5-phenyl-2,3-dihydro-1H-1-benzazepin-2-one are obtained.

Example 157

In the same manner as in Example 35 and using 3-amino-1-tert-butylaminocarbonylmethyl-5-phenyl-2,3-dihydro-1H-1-benzazepin-2-one eluted first in Example 156, (+)-N-(1-tert-butylaminocarbonylmethyl-2-oxo-5-phenyl-2,3-dihydro-1H-1-benzazepin-3-yl)-2-indolecarboxamide is obtained.

Example 158

In the same manner as in Example 152, two kinds of optically active compounds of 3-amino-1-diethylaminocarbonylmethyl-5-phenyl-2,3-dihydro-1H-1-benzazepin-2-one are obtained.

Example 159

In the same manner as in Example 35 and using 3-amino-1-diethylaminocarbonylmethyl-5-phenyl-2,3-dihydro-1H-1-benzazepin-2-one eluted first in Example 158, (+)-N-(1-diethylaminocarbonylmethyl-2-oxo-5-phenyl-2,3-dihydro-1H-1-benzazepin-3-yl)-2-indolecarboxamide is obtained.

Example 160

In the same manner as in Example 152, two kinds of optically active compounds of 3-amino-5-phenyl-1-(pyrrolidin-1-ylcarbonylmethyl)-2,3-dihydro-1H-1-benzazepin-2-one are obtained.

Example 161

In the same manner as in Example 35 and using 3-amino-5-phenyl-1-(pyrrolidin-1-ylcarbonylmethyl)-2,3-dihydro-1H-1-benzazepin-2-one eluted first in Example 160, (+)-N-(2-oxo-5-phenyl-1-(pyrrolidin-1-ylcarbonylmethyl)-2,3-dihydro-1H-1-benzazepin-3-yl)-2-indolecarboxamide is obtained.

Example 162

In the same manner as in Example 31 and using 3-amino-1-ethoxycarbonylmethyl-5-phenyl-2,3-dihydro-1H-1-benzazepin-2-one obtained in Example 45 and tert-butyl 3-aminophenylacetate, (-)-1-(3-tert-butoxycarbonylmethylphenyl)-3-(1-ethoxycarbonylmethyl-2-oxo-5-phenyl-2,3-dihydro-1H-1-benzazepin-3-yl)urea is obtained.

Example 163

(-)-1-(3-tert-Butoxycarbonylmethylphenyl)-3-(1-ethoxycarbonylmethyl-2-oxo-5-phenyl-2,3-dihydro-1H-1-benzazepin-3-yl)urea is dissolved in trifluoroacetic acid. The mixture is stirred for 8 hours and concentrated under reduced pressure to give (-)-1-(3-carboxymethylphenyl)-3-(1-ethoxycarbonylmethyl-2-oxo-5-phenyl-2,3-dihydro-1H-1-benzazepin-3-yl)urea.

Example 164

In the same manner as in Example 46 and using 3-amino-1-tert-butoxycarbonylmethyl-5-phenyl-2,3-dihydro-1H-1-benzazepin-2-one eluted later in Example 152, (-)-1-(1-tert-butoxycarbonylmethyl-2 oxo-5-phenyl-2,3-dihydro-1H-1-benzazepin-3-yl)-3-(3-methylphenyl)urea is obtained.

Example 165

In the same manner as in Example 46, 3-amino-5-phenyl-1-isopropoxycarbonylmethyl-2,3-dihydro-1H-1-benzazepin-2-one eluted later in Example 154, (-)-1-(3-methylphenyl)-3-(2-oxo-5-phenyl-1-isopropoxycarbonylmethyl-2,3-dihydro-1H-1-benzazepin-3-yl)urea is obtained.

Example 166

In the same manner as in Example 46 and using 3-amino-1-tert-butylaminocarbonylmethyl-5-phenyl-2,3-dihydro-1H-1-benzazepin-2-one eluted later in Example 156, (-)-1-(1-tert-butylaminocarbonylmethyl-2-oxo-5-phenyl-2,3-dihydro-1H-1-benzazepin-3-yl)-3-(3-methylphenyl)urea is obtained.

Example 167

In the same manner as in Example 46 and using 3-amino-5-phenyl-1-(pyrrolidin-1-ylcarbonylmethyl)-2,3-dihydro-1H-1-benzazepin-2-one eluted later in Example 160, (-)-1-(3-methylphenyl)-3-(2-oxo-5-phenyl-1-(pyrrolidin-1-ylcarbonylmethyl)-2,3-dihydro-1H-1-benzazepin-3-yl)urea is obtained.

Example 168

In the same manner as in Example 31 and using 3-amino-5-phenyl-1-(pyrrolidin-1-ylcarbonylmethyl)-2,3-dihydro-1H-1-benzazepin-2-one eluted later in Example 160, (-)-1-(2-methylpyridin-6-yl)-3-(2-oxo-5-phenyl-1-(pyrrolidin-1-ylcarbonylmethyl)-2,3-dihydro-1H-1-benzazepin-3-yl)urea is obtained.

Example 169

In the same manner as in Example 168, (-)-1-(2-methoxypyridin-5-yl)-3-(2-oxo-5-phenyl-1-(pyrrolidin-1-ylcarbonylmethyl)-2,3-dihydro-1H-1-benzazepin-3-yl)urea is obtained.

Example 170

In the same manner as in Example 46 and using 3-amino-1-diethylaminocarbonylmethyl-5-phenyl-2,3-dihydro-1H-1-benzazepin-2-one eluted later in Example 158, (-)-1-(1-diethylaminocarbonylmethyl-2-oxo-5-phenyl-2,3-dihydro-1H-1-benzazepin-3-yl)-3-(3-methylphenyl)urea is obtained.

Example 171

In the same manner as in Example 31 and using 3-amino-1-diethylaminocarbonylmethyl-5-phenyl-2,3-dihydro-1H-1-benzazepin-2-one eluted later in Example 158 and 3-amino-(1H-tetrazol-5-yl)-benzene which can be prepared by the method described in Japanese Patent Unexamined Publication No. 87838/1994, (-)-1-(1-diethylaminocarbonylmethyl-2-oxo-5-phenyl-2,3-dihydro-1H-1-benzazepin-3-yl)-3-(3-(1H-tetrazol-5-yl)phenyl)urea is obtained.

Example 172

In the same manner as in Example 171, (-)-1-(1-diethylaminocarbonylmethyl-2-oxo-5-phenyl-2,3-dihydro-1H-1-benzazepin-3-yl)-3-(4-(1H-tetrazol-5-yl)phenyl)urea is obtained.

Example 173

In the same manner as in Example 172 and using 3-amino-5-phenyl-1-(pyrrolidin-1-ylcarbonylmethyl)-2,3-dihydro-1H-1-benzazepin-2-one eluted later in Example 160, (-)-1-(2-oxo-5-phenyl-1-(pyrrolidin-1-ylcarbonylmethyl)-2,3-dihydro-1H-1-benzazepin-3-yl)-3-(3-(1H-tetrazol-5-yl)phenyl)urea is obtained.

In the same manner as in these Examples, the compounds shown in the following Tables are obtained.

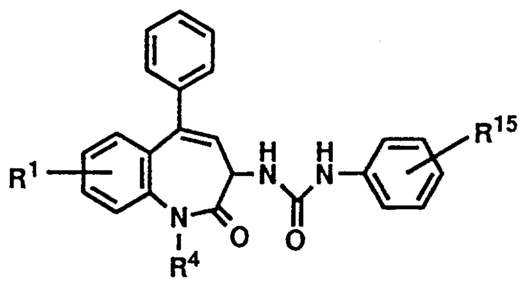

| No. | $R^1$ | $R^4$ | $R^{15}$ | configuration |
|---|---|---|---|---|
| 201 | H | $CH_3$ | 2-$CH_3$ | S |
| 202 | H | $CH_3$ | 3-$CH_3$ | S |
| 203 | H | $CH_3$ | 4-$CH_3$ | S |
| 204 | H | $CH_3$ | 4-$OCH_3$ | S |
| 205 | H | $CH_3$ | 3-$CH_2COOH$ | S |
| 206 | H | $CH_3$ | 2-$CH_3$ | R |
| 207 | H | $CH_3$ | 3-$CH_3$ | R |
| 208 | H | $CH_3$ | 4-$CH_3$ | R |
| 209 | H | $CH_3$ | 4-$OCH_3$ | R |
| 210 | H | $CH_3$ | 3-$CH_2COOH$ | R |

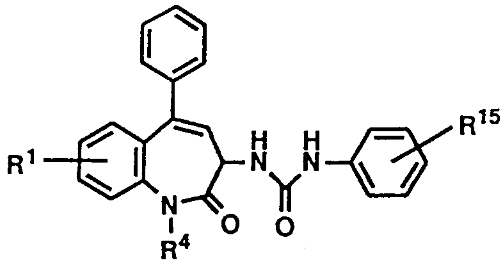

| No. | $R^1$ | $R^4$ | $R^{15}$ | configuration |
|---|---|---|---|---|
| 211 | H | 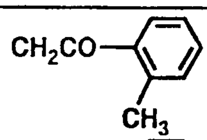 | 2-$CH_3$ | S |
| 212 | H | 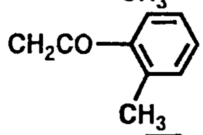 | 3-$CH_3$ | S |
| 213 | H | 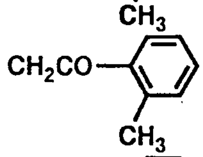 | 4-$CH_3$ | S |
| 214 | H | 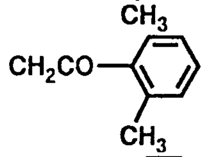 | 4-$OCH_3$ | S |
| 215 | H | 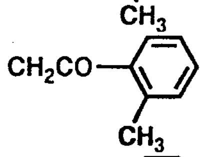 | 3-$CH_2COOH$ | S |
| 216 | H | 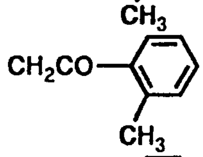 | 2-$CH_3$ | R |
| 217 | H | 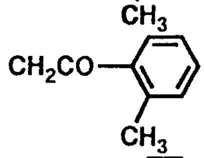 | 3-$CH_3$ | R |
| 218 | H | 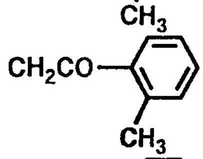 | 4-$CH_3$ | R |
| 219 | H | 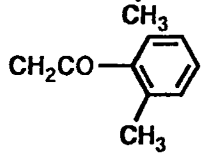 | 4-$OCH_3$ | R |
| 220 | H | 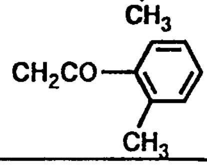 | 3-$CH_2COOH$ | R |

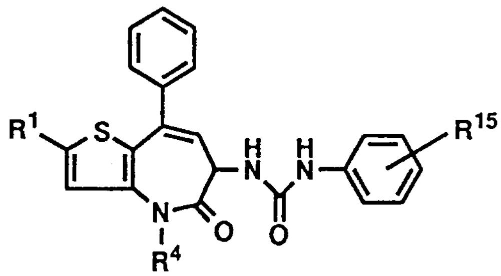

| No. | $R^1$ | $R^4$ | $R^{15}$ | configuration |
|---|---|---|---|---|
| 221 | $CH_3$ | $CH_3$ | 2-$CH_3$ | S |
| 222 | $CH_3$ | $CH_3$ | 3-$CH_3$ | S |
| 223 | $CH_3$ | $CH_3$ | 4-$CH_3$ | S |
| 224 | $CH_3$ | $CH_3$ | 4-$OCH_3$ | S |
| 225 | $CH_3$ | $CH_3$ | 3-$CH_2COOH$ | S |
| 226 | $CH_3$ | $CH_3$ | 2-$CH_3$ | R |
| 227 | $CH_3$ | $CH_3$ | 3-$CH_3$ | R |
| 228 | $CH_3$ | $CH_3$ | 4-$CH_3$ | R |
| 229 | $CH_3$ | $CH_3$ | 4-$OCH_3$ | R |
| 230 | $CH_3$ | $CH_3$ | 3-$CH_2COOH$ | R |

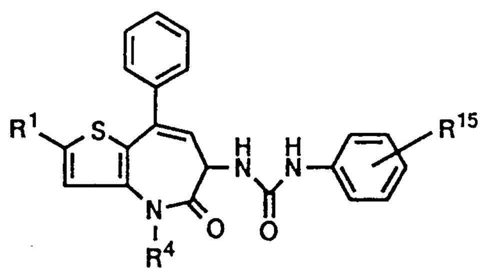

| No. | $R^1$ | $R^4$ | $R^{15}$ | configuration |
|---|---|---|---|---|
| 231 | $CH_3$ | 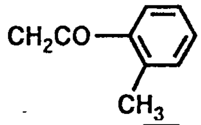 | $2-CH_3$ | S |
| 232 | $CH_3$ | 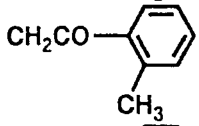 | $3-CH_3$ | S |
| 233 | $CH_3$ | 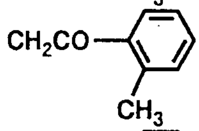 | $4-CH_3$ | S |
| 234 | $CH_3$ | 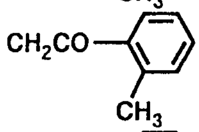 | $4-OCH_3$ | S |
| 235 | $CH_3$ | 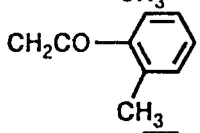 | $3-CH_2COOH$ | S |
| 236 | $CH_3$ | 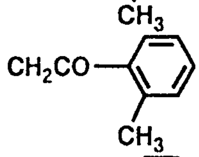 | $2-CH_3$ | R |
| 237 | $CH_3$ | 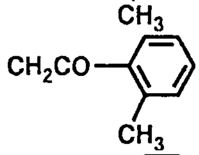 | $3-CH_3$ | R |
| 238 | $CH_3$ | 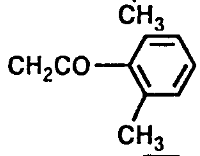 | $4-CH_3$ | R |
| 239 | $CH_3$ | 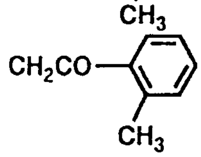 | $4-OCH_3$ | R |
| 240 | $CH_3$ | 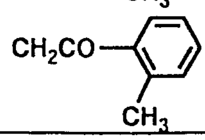 | $3-CH_2COOH$ | R |

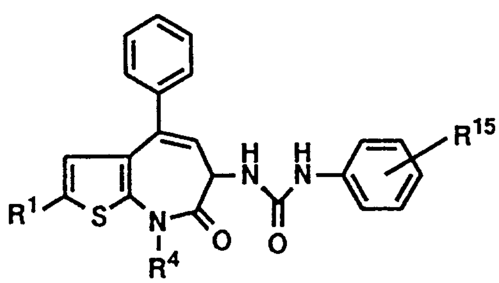

| No. | $R^1$ | $R^4$ | $R^{15}$ | configuration |
|---|---|---|---|---|
| 241 | $CH_3$ | $CH_3$ | 2-$CH_3$ | S |
| 242 | $CH_3$ | $CH_3$ | 3-$CH_3$ | S |
| 243 | $CH_3$ | $CH_3$ | 4-$CH_3$ | S |
| 244 | $CH_3$ | $CH_3$ | 4-$OCH_3$ | S |
| 245 | $CH_3$ | $CH_3$ | 3-$CH_2COOH$ | S |
| 246 | $CH_3$ | $CH_3$ | 2-$CH_3$ | R |
| 247 | $CH_3$ | $CH_3$ | 3-$CH_3$ | R |
| 248 | $CH_3$ | $CH_3$ | 4-$CH_3$ | R |
| 249 | $CH_3$ | $CH_3$ | 4-$OCH_3$ | R |
| 250 | $CH_3$ | $CH_3$ | 3-$CH_2COOH$ | R |

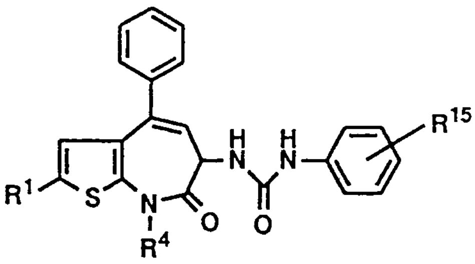

| No. | $R^1$ | $R^4$ | $R^{15}$ | configuration |
|---|---|---|---|---|
| 251 | $CH_3$ | 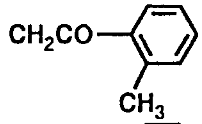 | 2-$CH_3$ | S |
| 252 | $CH_3$ | 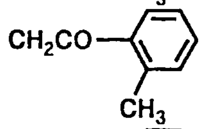 | 3-$CH_3$ | S |
| 253 | $CH_3$ | 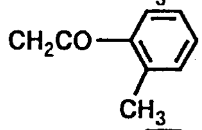 | 4-$CH_3$ | S |
| 254 | $CH_3$ | 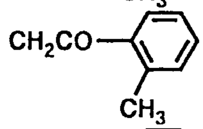 | 4-$OCH_3$ | S |
| 255 | $CH_3$ | 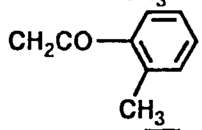 | 3-$CH_2COOH$ | S |
| 256 | $CH_3$ | 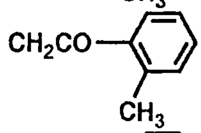 | 2-$CH_3$ | R |
| 257 | $CH_3$ | 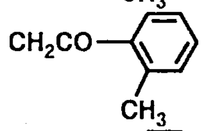 | 3-$CH_3$ | R |
| 258 | $CH_3$ | 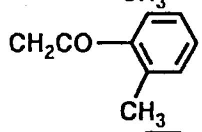 | 4-$CH_3$ | R |
| 259 | $CH_3$ | 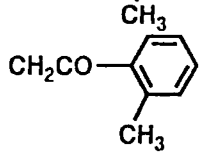 | 4-$OCH_3$ | R |
| 260 | $CH_3$ | 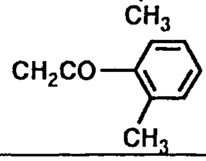 | 3-$CH_2COOH$ | R |

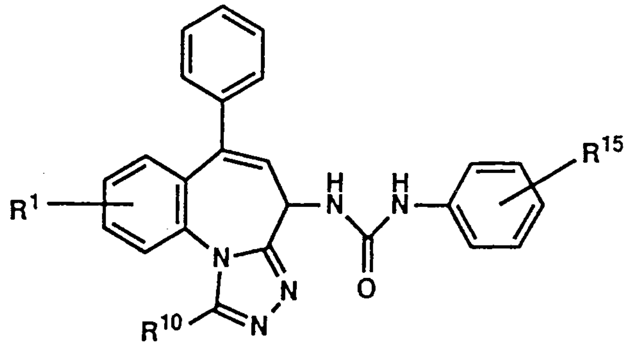

| No. | $R^1$ | $R^{10}$ | $R^{15}$ | configuration |
|---|---|---|---|---|
| 261 | H | 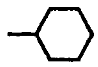 | 2-$CH_3$ | S |
| 262 | H | 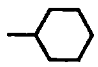 | 3-$CH_3$ | S |
| 263 | H | 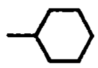 | 4-$CH_3$ | S |
| 264 | H | 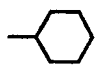 | 4-$OCH_3$ | S |
| 265 | H | 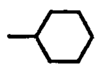 | 3-$CH_2COOH$ | S |
| 266 | H | 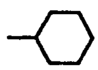 | 2-$CH_3$ | R |
| 267 | H | 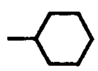 | 3-$CH_3$ | R |
| 268 | H | 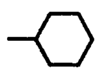 | 4-$CH_3$ | R |
| 269 | H | 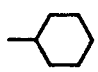 | 4-$OCH_3$ | R |
| 270 | H | 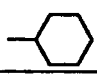 | 3-$CH_2COOH$ | R |

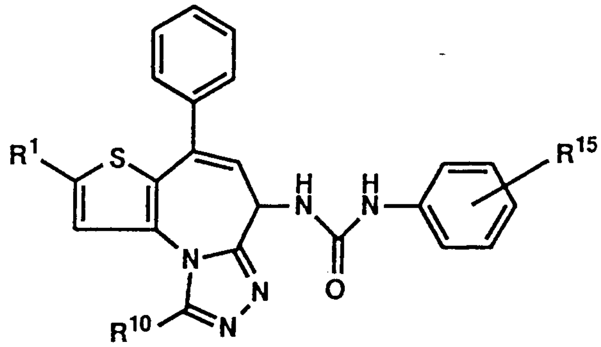

| No. | $R^1$ | $R^{10}$ | $R^{15}$ | configuration |
|---|---|---|---|---|
| 271 | $CH_3$ | 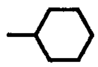 | 2-$CH_3$ | S |
| 272 | $CH_3$ | 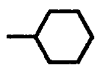 | 3-$CH_3$ | S |
| 273 | $CH_3$ | 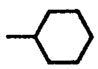 | 4-$CH_3$ | S |
| 274 | $CH_3$ | 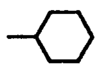 | 4-$OCH_3$ | S |
| 275 | $CH_3$ | 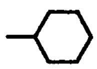 | 3-$CH_2COOH$ | S |
| 276 | $CH_3$ | 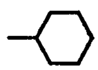 | 2-$CH_3$ | R |
| 277 | $CH_3$ | 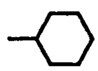 | 3-$CH_3$ | R |
| 278 | $CH_3$ | 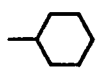 | 4-$CH_3$ | R |
| 279 | $CH_3$ | 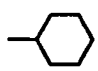 | 4-$OCH_3$ | R |
| 280 | $CH_3$ | 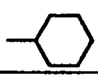 | 3-$CH_2COOH$ | R |

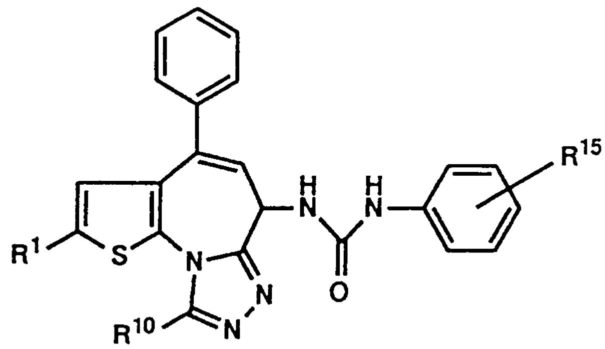

| No. | $R^1$ | $R^{10}$ | $R^{15}$ | configuration |
|---|---|---|---|---|
| 281 | $CH_3$ | 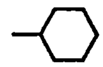 | $2\text{-}CH_3$ | S |
| 282 | $CH_3$ | 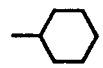 | $3\text{-}CH_3$ | S |
| 283 | $CH_3$ | 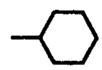 | $4\text{-}CH_3$ | S |
| 284 | $CH_3$ | 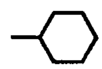 | $4\text{-}OCH_3$ | S |
| 285 | $CH_3$ | 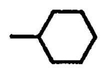 | $3\text{-}CH_2COOH$ | S |
| 286 | $CH_3$ | 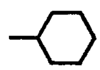 | $2\text{-}CH_3$ | R |
| 287 | $CH_3$ | 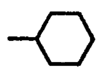 | $3\text{-}CH_3$ | R |
| 288 | $CH_3$ | 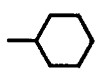 | $4\text{-}CH_3$ | R |
| 289 | $CH_3$ | 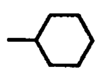 | $4\text{-}OCH_3$ | R |
| 290 | $CH_3$ | 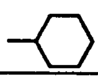 | $3\text{-}CH_2COOH$ | R |

<u>Formulation Example 1</u>

The compound of Example 46 (0.5 part), lactose (25 parts), crystalline cellulose (35 parts) and corn starch (3 parts) were thoroughly admixed, and kneaded well with a binder prepared from corn starch (2 parts). The kneaded substance was passed through a 16 mesh sieve, dried in an oven at 50°C and passed through a 24 mesh sieve. The thus obtained kneaded powder, corn starch (8 parts), crystalline cellulose (11 parts) and talc (9 parts) were thoroughly mixed and compressed to give tablets containing 0.5 mg of the effective ingredient per tablet.

Formulation Example 2

The compound of Example 46 (1.0 mg) and sodium chloride (9.0 mg) were dissolved in water for injection and filtered to remove pyrogen. The filtrate was moved aseptically into ampoules, sterilized and melt-sealed to give an injection containing 1.0 mg of the effective ingredient.

Industrial Applicability

The compound of the present invention has superior cholecystokinin antagonistic action and gastrin antagonistic action, and shows strong and long-lasting pancreatic enzyme inhibitory action and gastric acid secretion inhibitory action. Hence, the compound is useful as a therapeutic agent acting on the central and peripheral nerves, in particular, as a therapeutic agent for various central nervous diseases such as anxiety, depression and schizofrenia, or as an agent for the prophylaxis and treatment of pancreatic disorders and gastrointestinal ulcers. In addition, the compound of the present invention is expected to have anti-demantia action based on the cholecystokinin antagonistic action and is useful as an antidementia.

**Claims**

**1.** A 3-aminoazepine compound of the formula

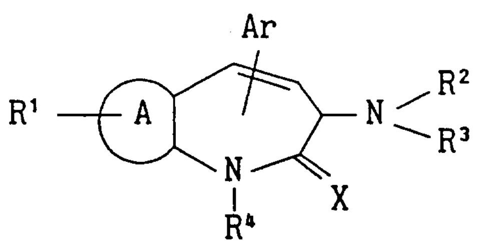

(I)

wherein

Ar is an optionally substituted aryl or an optionally substituted heteroaryl;

ring A is a benzene, a thiophene or a pyridine;

$R^1$ is a hydrogen, an alkyl, a cycloalkyl, a cycloalkylalkyl, an aralkyl, a hydroxy, an alkoxy, an amino, an alkylamino, a halogen or a group of the formula

$$-(CH_2)_kCOR^5$$

wherein k is 0 or an integer of 1-6 and $R^5$ is hydrogen, alkyl, cycloalkyl, cycloalkylalkyl, optionally substituted aryl, optionally substituted heteroaryl, aralkyl, hydroxy, alkoxy, amino, alkylamino, arylamino or aralkylamino;

$R^2$ and $R^3$ are the same or different and each is a hydrogen, alkyl, cycloalkyl, cycloalkylalkyl, aralkyl, a group of the formula

$$-(CH_2)_lC(=Y)-R^6$$

wherein l is 0 or an integer of 1-6, Y is oxygen atom or sulfur atom, $R^6$ is hydrogen, alkyl, cycloalkyl, cycloalkylalkyl, optionally substituted aryl, optionally substituted heteroaryl, aralkyl, hydroxy, alkoxy or amino, a group of the formula

$$-C(=Y)-NH-R^7$$

wherein Y is oxygen atom or sulfur atom, $R^7$ is alkyl, cycloalkyl, cycloalkylalkyl, optionally substituted aryl, optionally substituted heteroaryl or aralkyl; or a group of the formula

$$-(CH_2)_mS(O)_n-R^8$$

wherein m is 0 or an integer of 1-6, n is 0, 1 or 2 and $R^8$ is alkyl, cycloalkyl, cycloalkylalkyl, optionally substituted aryl, optionally substituted heteroaryl, aralkyl, hydroxy, alkoxy, amino, alkylamino, arylamino or aralkylamino, or $-N(R^2)(R^3)$ may be an amino group protected by a protecting group;

$R^4$ is a hydrogen, an alkyl, a cycloalkyl, a cycloalkylalkyl, an alkenyl, an aralkyl or a group of the formula

$$-(CH_2)_pCOR^9$$

wherein p is 0 or an integer of 1-6 and $R^9$ is hydrogen, alkyl, cycloalkyl, cycloalkylalkyl, optionally substituted aryl, optionally substituted heteroaryl, aralkyl, hydroxy, alkoxy, amino, alkylamino, arylamino, aralkylamino, or a cyclic amino of the formula

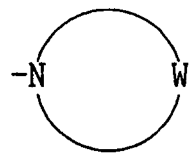

(a)

wherein W is $CH_2$, O or $N-R^{15}$ where $R^{15}$ is hydrogen, alkyl, acyl or benzyl; and

X is an oxygen atom or a sulfur atom, or $R^4$ and X are bonded to each other to form a group of the formula $-C(R^{10})=N-N=$ where $R^{10}$ is hydrogen, alkyl, alkenyl, cycloalkyl, cycloalkylalkyl, adamantyl, aryl, aralkyl or a group of the formula

$$-(CH_2)_qCOR^{11}$$

wherein q is 0 or an integer of 1-6 and $R^{11}$ is hydrogen, alkyl, cycloalkyl, cycloalkylalkyl, aryl, heteroaryl, aralkyl, hydroxy, alkoxy, amino, alkylamino, arylamino or aralkylamino,

an optically active compound thereof or a pharmaceutically acceptable salt thereof.

**2.** The 3-aminoazepine compound of Claim 1, wherein the compound of the formula (I) is the compound of the formula

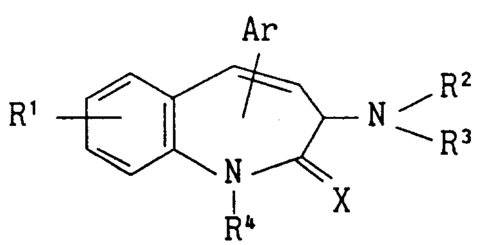

(Ia)

wherein each symbol is as defined in Claim 1 above,
an optically active compound thereof or a pharmaceutically acceptable salt thereof.

**3.** The 3-aminoazepine compound of Claim 1, wherein the compound of the formula (I) is the compound of the formula

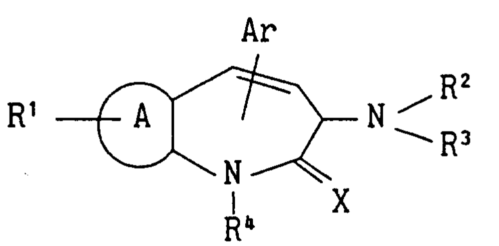

(Ib)

wherein A is a thiophene or a pyridine, and other symbols are as defined in Claim 1 above,
an optically active compound thereof or a pharmaceutically acceptable salt thereof.

**4.** The 3-aminoazepine compound of Claim 1, wherein the compound of the formula (I) is the compound of the formula

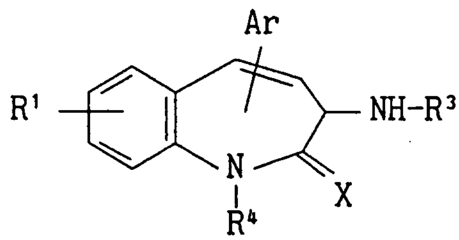

(Ic)

wherein

Ar is an optionally substituted phenyl;
$R^1$ is a hydrogen or an alkyl;
$R^3$ is a group of the formula

$$-(CH_2)_lC(=Y)-R^6$$

wherein l is 0 or an integer of 1-6, Y is oxygen atom or sulfur atom, $R^6$ is hydrogen, alkyl, cycloalkyl, cycloalkylalkyl, optionally substituted aryl, optionally substituted heteroaryl, aralkyl, hydroxy, alkoxy or amino, or a group of the formula

$$-C(=Y)-NH-R^7$$

wherein Y is oxygen atom or sulfur atom, $R^7$ is alkyl, cycloalkyl, cycloalkylalkyl, optionally substituted aryl, optionally substituted heteroaryl or aralkyl;
$R^4$ is a hydrogen, an alkyl or a group of the formula

$$-(CH_2)_pCOR^9$$

wherein p is an integer of 1-6 and $R^9$ is alkyl, cycloalkyl, cycloalkylalkyl, optionally substituted aryl, optionally substituted heteroaryl, aralkyl, hydroxy, alkoxy, amino, alkylamino, arylamino, aralkylamino, or a cyclic amino of the formula

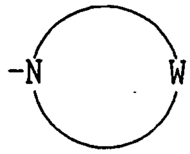

(a)

wherein W is $CH_2$, O or N-$R^{15}$ where $R^{15}$ is hydrogen, alkyl, acyl or benzyl; and
X is an oxygen atom, or $R^4$ and X are bonded to each other to form a group of the formula: $-C(R^{10})=N-N=$ where $R^{10}$ is alkyl or cycloalkyl,

an optically active compound thereof or a pharmaceutically acceptable salt thereof.

5. The 3-aminoazepine compound of Claim 1, wherein the compound of the formula (I) is the compound of the formula

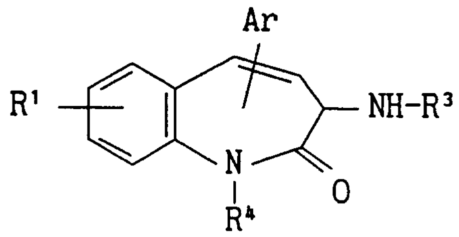

(Id)

wherein

Ar is an optionally substituted phenyl,
$R^1$ is a hydrogen or an alkyl;
$R^3$ is a group of the formula

$$-(CH_2)_lC(=Y)-R^6$$

wherein l is 0, Y is oxygen atom, $R^6$ is hydrogen, alkyl, cycloalkyl, cycloalkylalkyl, optionally substituted aryl, optionally substituted heteroaryl, aralkyl, hydroxy, alkoxy or amino, or a group of the formula

$$-C(=Y)-NH-R^7$$

wherein Y is oxygen atom, $R^7$ is alkyl, cycloalkyl, cycloalkylalkyl, optionally substituted aryl, optionally substituted heteroaryl or aralkyl; and
$R^4$ is an alkyl or a group of the formula

$$-(CH_2)_pCOR^9$$

wherein p is 1 and $R^9$ is optionally substituted aryl, optionally substituted heteroaryl, hydroxy, alkoxy, amino, alkylamino, arylamino, aralkylamino, or a cyclic amino of the formula

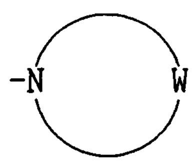

(a)

wherein W is $CH_2$, O or N-$R^{15}$ where $R^{15}$ is hydrogen, alkyl, acyl or benzyl,

an optically active compound thereof or a pharmaceutically acceptable salt thereof.

6. The 3-aminoazepine compound of Claim 1, wherein the compound of the formula (I) is the compound of the formula

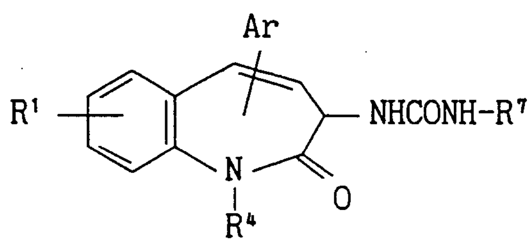

(Ie)

wherein

Ar is an optionally substituted phenyl;

$R^1$ is a hydrogen;

$R^7$ is an optionally substituted aryl or an optionally substituted heteroaryl; and

$R^4$ is a group of the formula

$$-(CH_2)_pCOR^9$$

wherein p is 1 and $R^9$ is optionally substituted aryl, hydroxy, alkoxy, alkylamino, or a cyclic amino of the formula

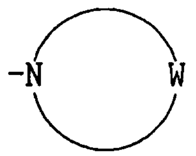

(a)

wherein W is $CH_2$, O or N-$R^{15}$ where $R^{15}$ is hydrogen, alkyl, acyl or benzyl,

an optically active compound thereof or a pharmaceutically acceptable salt thereof.

**7.** The compound of Claim 1, wherein the compound of the formula (I) is a compound selected from the group consisting of:
(+)-N-(1-methyl-2-oxo-5-phenyl-2,3-dihydro-1H-1-benzazepin-3-yl)-2-indolecarboxamide,
(+)-N-(1-ethoxycarbonylmethyl-2-oxo-5-phenyl-2,3-dihydro-1H-1-benzazepin-3-yl)-2-indolecarboxamide,
(+)-N-(1-(2'-methylphenacyl)-2-oxo-5-phenyl-2,3-dihydro-1H-1-benzazepin-3-yl)-2-indolecarboxamide,
(+)-N-(1-tert-butoxycarbonylmethyl-2-oxo-5-phenyl-2,3-dihydro-1H-1-benzazepin-3-yl)-2-indolecarboxamide,
(+)-N-(1-isopropoxycarbonylmethyl-2-oxo-5-phenyl-2,3-dihydro-1H-1-benzazepin-3-yl)-2-indolecarboxamide,
(+)-N-(1-tert-butylaminocarbonylmethyl-2-oxo-5-phenyl-2,3-dihydro-1H-1-benzazepin-3-yl)-2-indolecarboxamide,
(+)-N-(1-diethylaminocarbonylmethyl-2-oxo-5-phenyl-2,3-dihydro-1H-1-benzazepin-3-yl)-2-indolecarboxamide,
and
(+)-N-(2-oxo-5-phenyl-1-(pyrrolidin-1-ylcarbonylmethyl)-2,3-dihydro-1H-1-benzazepin-3-yl)-2-indolecarboxamide,
or a pharmaceutically acceptable salt thereof.

**8.** The compound of Claim 1, wherein the compound of the formula (I) is a compound selected from the group consisting of:
(-)-1-(1-ethoxycarbonylmethyl-2-oxo-5-phenyl-2,3-dihydro-1H-1-benzazepin-3-yl)-3-(3-methylphenyl)urea,
(-)-1-(1-ethoxycarbonylmethyl-2-oxo-5-phenyl-2,3-dihydro-1H-1-benzazepin-3-yl)-3-(4-methylphenyl)urea,
(-)-1-(1-ethoxycarbonylmethyl-2-oxo-5-phenyl-2,3-dihydro-1H-1-benzazepin-3-yl)-3-(3-methoxyphenyl)urea,
(-)-1-(1-(2'-methylphenacyl)-2-oxo-5-phenyl-2,3-dihydro-1H-1-benzazepin-3-yl)-3-(4-methylphenyl)urea,
(-)-1-(1-(2'-methylphenacyl)-2-oxo-5-phenyl-2,3-dihydro-1H-1-benzazepin-3-yl)-3-(2-methylphenyl)urea,
(-)-1-(4-methoxyphenyl)-3-(1-(2'-methylphenacyl)-2-oxo-5-phenyl-2,3-dihydro-1H-1-benzazepin-3-yl)urea,
(-)-1-(2-chlorophenyl)-3-(1-(2'-methylphenacyl)-2-oxo-5-phenyl-2,3-dihydro-1H-1-benzazepin-3-yl)urea,
(-)-1-(3-carboxymethylphenyl)-3-(1-(2'-methylphenacyl)-2-oxo-5-phenyl-2,3-dihydro-1H-1-benzazepin-3-yl)urea,
(-)-1-(3-carboxymethylphenyl)-3-(1-ethoxycarbonylmethyl-2-oxo-5-phenyl-2,3-dihydro-1H-1-benzazepin-3-yl)urea,
(-)-1-(1-tert-butoxycarbonylmethyl-2-oxo-5-phenyl-2,3-dihydro-1H-1-benzazepin-3-yl)-3-(3-methylphenyl)urea,
(-)-1-(3-methylphenyl)-3-(2-oxo-5-phenyl-1-isopropoxycarbonylmethyl-2,3-dihydro-1H-1-benzazepin-3-yl)urea,
(-)-1-(1-tert-butylaminocarbonylmethyl-2-oxo-5-phenyl-2,3-dihydro-1H-1-benzazepin-3-yl)-3-(3-methylphenyl)urea,
(-)-1-(3-methylphenyl)-3-(2-oxo-5-phenyl-1-(pyrrolidin-1-ylcarbonylmethyl)-2,3-dihydro-1H-1-benzazepin-3-yl)urea,
(-)-1-(2-methylpyridin-6-yl)-3-(2-oxo-5-phenyl-1-(pyrrolidin-1-ylcarbonylmethyl)-2,3-dihydro-1H-1-benzazepin-3-yl)urea,
(-)-1-(2-methoxypyridin-5-yl)-3-(2-oxo-5-phenyl-1-(pyrrolidin-1-ylcarbonylmethyl)-2,3-dihydro-1H-1-benzazepin-3-yl)urea,
(-)-1-(1-diethylaminocarbonylmethyl-2-oxo-5-phenyl-2,3-dihydro-1H-1-benzazepin-3-yl)-3-(3-methylphenyl)urea,
(-)-1-(1-diethylaminocarbonylmethyl-2-oxo-5-phenyl-2,3-dihydro-1H-1-benzazepin-3-yl)-3-(3-(1H-tetrazol-5-yl)phenyl)urea,
(-)-1-(1-diethylaminocarbonylmethyl-2-oxo-5-phenyl-2,3-dihydro-1H-1-benzazepin-3-yl)-3-(4-(1H-tetrazol-5-yl)phenyl)urea, and

(-)-1-(2-oxo-5-phenyl-1-(pyrrolidin-1-ylcarbonylmethyl)-2,3-dihydro-1H-1-benzazepin-3-yl)-3-(3-(1H-tetrazol-5-yl)phenyl)urea, or a pharmaceutically acceptable salt thereof.

**9.** A pharmaceutical composition comprising the compound of Claim 1 and a carrier for pharmaceuticals.

**10.** A cholecystokinin antagonist comprising the compound of Claim 1.

## INTERNATIONAL SEARCH REPORT

International application No.
PCT/JP94/00774

**A. CLASSIFICATION OF SUBJECT MATTER**

Int. $Cl^5$ C07D223/16, C07D401/00, C07D403/00, C07D471/00, C07D487/00, C07D495/00, C07K5/00, A61K31/55, A61K37/02

According to International Patent Classification (IPC) or to both national classification and IPC

**B. FIELDS SEARCHED**

Minimum documentation searched (classification system followed by classification symbols)

Int. $Cl^5$ C07D223/16, C07D401/00, C07D403/00, C07D471/00, C07D487/00, C07D495/00, C07K5/00, A61K31/55, A61K37/02

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| E, A | JP, A, 6-172316 (Merck & Co., Inc.), June 21, 1994 (21. 06. 94), (Family: none) | 1-10 |
| T, A | JP, A, 6-128257 (Yoshitomi Pharmaceutical Co., Ltd.), May 10, 1994 (10. 05. 94), (Family: none) | 1-10 |
| A | JP, A, 5-105667 (Merck & Co., Inc.), April 27, 1993 (27. 04. 93) & US, A, 5206234 & EP, A, 487207 | 1-10 |
| A | JP, A, 3-223290 (Yoshitomi Pharmaceutical Co., Ltd.), October 2, 1991 (02. 10. 91), (Family: none) | 1-10 |
| A | JP, A, 2-243691 (Boehringer Ingelheim KG.), September 27, 1990 (27. 09. 90) & DE, A, 3936828 & EP, A, 368175 | 1-10 |
| A | JP, A, 61-15874 (Merck & Co., Inc.), January 23, 1986 (23. 01. 86) | 1-10 |

[X] Further documents are listed in the continuation of Box C.  [ ] See patent family annex.

* Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| June 30, 1994 (30. 06. 94) | July 19, 1994 (19. 07. 94) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP94/00774

C (Continuation). DOCUMENTS CONSIDERED TO BE RELEVANT

| Category* | Citation of document, with indication, where appropriate, of the relevant passages | Relevant to claim No. |
|---|---|---|
| | & US, A, 4652641 & EP, A, 166353 | |

Form PCT/ISA/210 (continuation of second sheet) (July 1992)